# EUROPEAN PATENT APPLICATION

(11) **EP 2 028 271 A1**
(43) Date of publication of application: **25.02.2009**
(21) Application number: 07114785.4
(22) Date of filing: 22.08.2007
(51) Int. Cl.: C12N 9/80, C12N 15/62

(54) **New proteins for use in human and animal staphylococcus infections**

(71) Applicant: Profos AG, 93053 Regensburg (DE)
(72) Inventor: Forchheim, Michael, 93053, Regensburg (DE); Grallert, Holger, 93053, Regensburg (DE); Philipp, Anja, 93051, Regensburg (DE); Biebl, Manfred, 93053, Regensburg (DE)
(74) Representative: Dehmel, Albrecht

(57) **Abstract**

The present invention relates to a polypeptide termed ply_pitti26 comprising the sequence as depicted in SEQ ID NO:1 as well as variants of this polypeptide. Furthermore, the present invention relates to nucleic acids and vectors encoding for said polypeptide and variants thereof as well as host cells comprising these nucleic acids and/or vectors. Finally, the present invention relates to the uses of said polypeptide, variants thereof, nucleic acid sequences, vectors and host cells, in particular for the treatment or prophylaxis of a subject infected by or exposed to Staphylococci.

## Description

The present invention relates to a polypeptide termed ply_pitti26 comprising the sequence as depicted in SEQ ID NO:1 as well as variants of this polypeptide. Furthermore, the present invention relates to nucleic acids and vectors encoding for said polypeptide and variants thereof as well as host cells comprising these nucleic acids and/or vectors. Finally, the present invention relates to the uses of said polypeptide, variants thereof, nucleic acid sequences, vectors and host cells, in particular for the treatment or prophylaxis of a subject infected by or exposed to Staphylococci.

### Background of the Invention

Staphylococcal infections are a major cause of severe diseases with high mortality all over the world. The gram-positive pathogen *Staphylococcus aureus* is responsible for a variety of infections of the skin and soft tissues as well as life-threatening infections like bacteremia and endocarditis. In addition, *Staphylococcus aureus* is frequently involved in food poisoning. Due to its tolerance to low pH values and high salt conditions this pathogen grows in a variety of food products, especially of animal origin, producing a heat stable enterotoxin. Persons with a particular risk of infection are patients after surgery or during hemodialysis as well as premature infants and immunocompromised persons, or those with need for prosthetic devices. Staphylococcal infections are of particular global health concern because of their high distribution (about 25 - 30 % of the population are asymptomatic carriers) and of the increasing emergence of antibiotic resistant strains of *Staphylococcus aureus.* MRSA (methicillin resistant *Staphylococcus aureus*) is a prominent member of this group and a major cause of nosocomial infections. In addition, there are many multiresistant strains, even those which are resistant to the "drugs of the last line of defence" like vancomycin, linezolid or daptomycin. Infections with antibiotic resistant staphylococci rise enormous consts to the global health budgets, because the patients often need long-term stay in a hospital and have to be isolated from other patients.

Besides the coagulase positive *S. aureus,* pathogens from the group of the coagulase negative staphylococci are of importance. *S. haemolyticus*, for example, causes keratitis, *S. epidermidis* is frequent found in biofilms on implanted devices which are associated with serious infections (endoplastitis) and *S. saprophyticus* is responsible for urinary tract infections. Apart from infections of humans, cattle infections also play an important role. Especially, bovine mastitis, an infection of the mammary glands, is of commercial significance. Apart from *S. aureus* it is caused by some coagulase negative staphylococci like *S. epidermidis, S. simulans, S. chromogenes, S. hyicus, S. warneri* and *S. xylosus.*

Standard antibiotic therapy is becoming more and more ineffective. Hence, new strategies for treating bacterial infections are needed. They include the development of new antibiotics as well as the search for antimicrobial peptides. Uses of antibodies and putative vaccines or phage therapy are alternative approaches. However all of these methods exhibit serious disadvantages. At widespread use, novel antibiotics also will rise new resistances, antimicrobial peptides and monoclonal antibodies require a lot of additional investments until a routine use in therapy will be possible; immunization strategies against *Staphylococcus aureus* were not successful so far and phage therapy causes problems with immune response and tissue penetration as well as with a potential undesired transfer of bacterial toxins by the phages. The use of isolated peptidoglycan hydrolases, the so-called endolysins, represents an advancement of the phage therapy. Endolysins enzymatically hydrolyse the cell walls of those bacteria which are host organisms for their corresponding bacteriophages.

### Description of the Relevant Art

After infection of the host bacterium, bacteriophages produce new phage particles within the host cell. At the end of the reproduction cycle the host cell must be lysed, to set free the new phage generation. Endolysins are produced as a tool for this lysis of the host cell. It was found, that endolysins also act on bacterial cell walls when they are added exogenously to non infected bacterial cells ("lysis from without"). The use of endolysins to kill contaminating bacteria in food was first disclosed by Gasson in 1991 (GB 2,255,561). First therapeutic and prophylactic applications *in vivo* using mouse model systems were described in 2001 by the group of Fischetti (Nelson & Fischetti, 2001, Proc. Natl. Acad. Sci. USA, 98, 4107-4112; Loeffler et al., 2001, Science, 94, 2170-2172). This work describes the topical application of endolysins against group A streptococci (oral application) and against pneumococci (nasopharyngeal application). Later, an application against *Bacillus anthracis* was added (Schuch et al., 2002, Nature 418, 884-889). Entenza et al. (2005, Antimicrob. Agents Chemother., 49, 4789-4792) report the use of Cpl-1 lysin against pneumococci causing endocarditis in rats. Endolysin PlyGBS was used to kill group B streptococci in the vagina and oropharynx of a mouse model (Cheng et al., 2005, Antimicrob.
Agents Chemother., 49, 111-117). Fischetti (2006, BMC Oral Health, 6, 16-19) summarizes the use of phage lytic enzymes to control pathogenic bacteria.

In US 5,997,862 a multitude of methods of treatments and pharmaceutical compositions to treat and prevent bacterial infections using phage derived lysins is disclosed. Several further patents teach specific compositions and uses of phage derived lysins for treatment of e.g. dermatological infections, ocular infections, infections of mouth and teeth, infections of the respiratory tract, various illnesses, bacterial infections in general, the parenteral use of lysin compositions, and the use of bandage compositions. US 2007-077235 describes lysin compositions to treat mastitis in animals.

Endolysins may be divided into five classes: (1) N-acetylmuramidases (lysozymes), (2) endo-β-N-acetylglucosaminidases, and (3) lytic transglycosylases, which all cleave the sugar moiety of peptidoglycan, (4) endopeptidases, which cleave the peptide moiety, and (5) N-actylmuramoyl-L-alanine amidases, which cut the amide bond between sugar backbone and peptide linkers. Endolysins show a modular organization exhibiting a combination of different polypeptide domains showing enzymatic activity or cell binding activity, the so-called EADs (enzymatically active domains) and CBDs (cell binding domains), respectively. Mostly, EADs are located at the N-terminal part of the endolysins, and CBDs at the C-terminal parts, but there are also exceptions of this rule of thumb. It is also shown that modules can be exchanged between different cell wall lytic enzymes producing new functional enzymes, which sometimes exhibit even new functional properties (Diaz et al., 1990, Proc. Natl. Acad. Sci. USA, 87, 8125-8129; Croux et al., 1993, Molecular Microbiology, 9, 1019-1025; Donovan et al., 2006, Appl. & Environm. Microbiol., 72, 2988-2996).

Since endolysins are typically more specific than antibiotics, it is unlikely that resistance development will rapidly occur. Therefore, the use of suitable endolysins acting on *staphylococcus* bacteria is a desirable means for the fight against the respective infections. Several endolysins active against *staphylococcus* bacteria are already described in the relevant art. Protein 17 associated with phage P68 is a staphylococcal endolysin which exhibits antimicrobial activity also against clinical *S. aureus* isolates (Takac et al., 2005, Antimicrobial Agents and Chemotherapy, 49, 2934-2940). The endolysin plyTW derived from the *S. aureus* phage Twort needs only the N-terminal enzymatically active fragment for hydrolytic activity against bacterial cells, whereas the C-terminal part with homology to lysostaphin seems dispensable (Loessner et al., 1998, FEMS Microbiol. Lett, 162, 265-274). Donovan et al. (2006, Appl. & Environm. Microbiol., 72, 2988-2996) created a chimaeric endolysin between *Streptococcus agalactiae* B30 endolysin and lysostaphin of *Staphylococcus simulans* with potential use in treatment of mastitis. Several groups used the endolysin of *Staphylcoccus aureus* bacteriophage phi11 in antimicrobial applications. Navarre et al (1999, J. Biol. Chem., 274, 15847-15856) identified multiple enzymatic activities in phi11 endolysin, and showed that a mutant with deletion of the amidase domain is still active. Donovan et al. (2006, FEMS Microbiol. Lett, 265, 133-139) used complete phi11 endolysin as well as C-terminally truncated versions in assays against mastitis pathogens. Different mutants of phi11 endolysin and phi12 endolysin were tested in different activity assays on *Staphylococcus aureus* cell walls, heat inactivated cells and also bacterial biofilms (Sass & Bierbaum, 2007, Appl. & Environment. Microbiol., 73, 347-352). The endolysin of the *Staphylococcus warneri* phage ΦWMY, LysWMY, although reported to be closely related to phi11 endolysin, retained full activity when the amidase as well as the cell binding domains were deleted (Yokoi et al., 2005, Gene 351, 97-108). This result indicates that the functions of and interactions between the different endolysin modules are not equivalent even in closely related endolysins.

Although different endolysins against staphylococcus bacteria are known from the art, there is still a need for efficient staphylococcal endolysins that can be produced in an efficient way and in addition show high activity against microorganisms of the genus Staphylococcus.

The problem is solved by the subject matter as disclosed in the claims.

### Brief Description of the Drawings

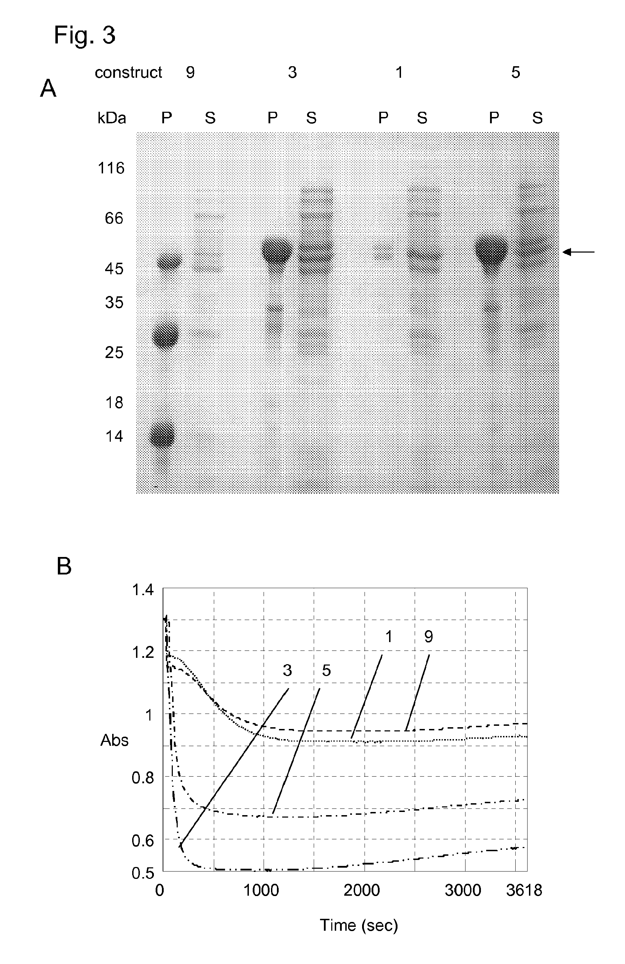

To illustrate certain aspects of the invention, the following drawings are presented. They are not intended to restrict the invention in any way.

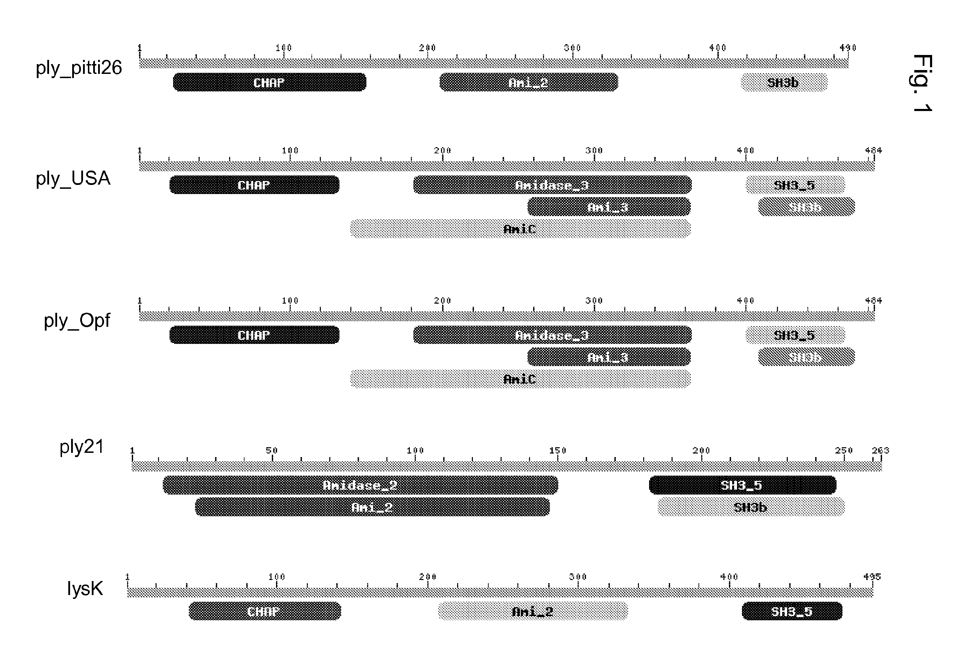

**Fig. 1** provides a schematic representation of the modular organization of different *Staphylococcus* endolysins
   The endolysins are built up from CHAP (cysteine, histidine-dependent amidohydrolases/peptidases), and amidase (N-acetyl-muramyl-L-alanine amidase, ami) enzymatic domains, and SH3-modules as cell binding domains (CBDs).

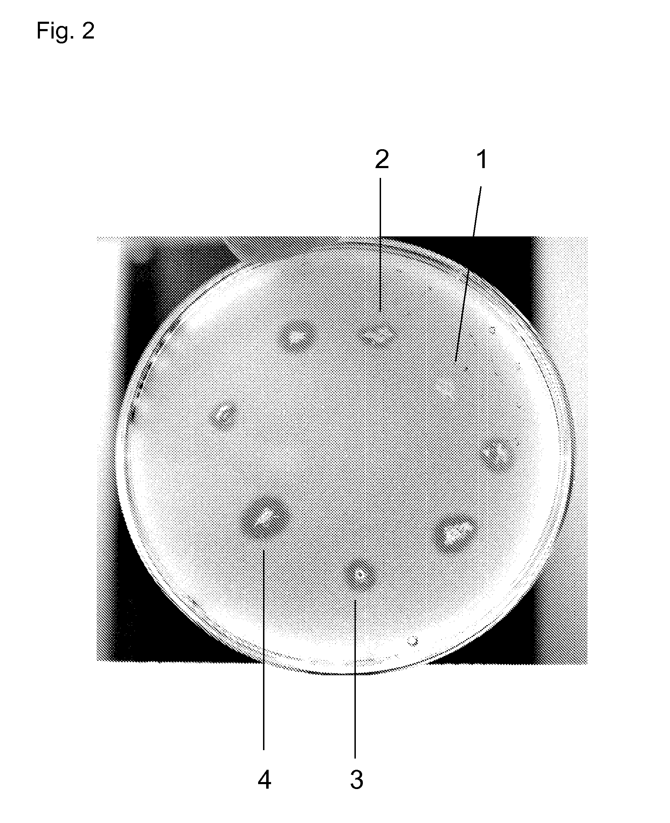

**Fig. 2** Agar plate showing lysis zones in an acitivity test on heat inactivated staphylococcus cells Lysis plates include the respective *staphylococcus* strain to be tested in a top agar layer. Either E. *coli* strains harbouring plasmids of the endolysin constructs to be tested or preparations of isolated endolysins are stippled to the top of the plates. Lysis zones appear after incubation depending on the lysing activity on the respective host bacterium. Number 1 depicts an example, where no lysis occurred (-). Numbers 2, 3, and 4 mark weak (+), medium (++) or strong (+++) lytic acticity of the respective endolysin variant. The agar plate in the figure exemplarily shows an assay, where the activity of several point mutations in EADplypitti26_CBDplyUSA were tested.
**Fig. 3** Comparison of different artificial staphylococcus endolysin constructs
   Artificial endolysin constructs according to the present invention are shown in this figure with respect to expression and solubility (fig. 3A), and activity in the turbidity assay (fig. 3B). Construct 3 is a synonym for EADplypitti26_CBDplyUSA, construct 5 is a synonym for EADplypitti26_CBDplypitti20, construct 9 and 5 are further artificial endolysins. The lanes on the SDS-gel are marked "P" for unsoluble pellet fraction and "S" for soluble supernatant fraction. A molecular mass standard is indicated in the margin. The position of the full-length endolysin constructs is marked by an arrow. The expression and solubility test at 37°C is performed as described in example 2, the activity test is done like in example 5, but with a raw cell extract after expression of the proteins.

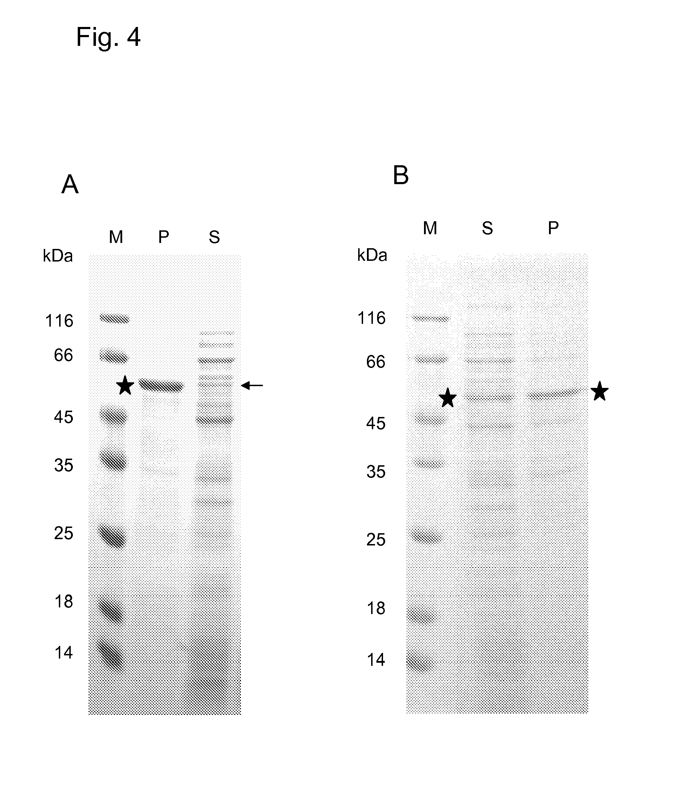

**Fig. 4** Comparison of solubility after expression
   Depicted are solubility tests performed after expression of endolysin constructs at 30°C (described in example 2). Fig. 4A shows an SDS-gel with the solubility test of wild-type plypitti26, fig. 4B a respective test with EADplypitti26_CBDplyUSA. "P" denotes the insoluble "pellet fraction" whereas "S" denotes the soluble protein fraction found in the supernatant. The bands of the endolysins are marked with asterics, the arrow in fig. 4A marks an *E. coli* protein which migrates only somewhat slower than plypitti26. The molecular mass marker lane is marked by an "M".

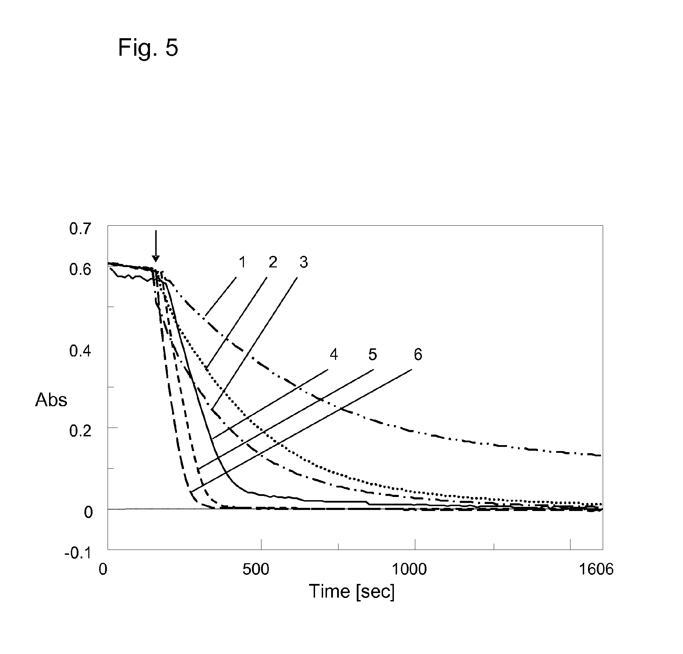

**Fig. 5** Comparison between lysis activity of EADplypitti26_CBDplyUSA and wild-type plypitti26 on untreated cells
   Concentration dependent lysis profiles were recorded on *Staphylococcus aureus* cells after addition of purified plypitti26 (numbers 1, 2, 3) or EADplypitti26_CBDplyUSA(numbers 4, 5, 6). The assay was performed as described in example 5. 2 µg, 5 µg or 10 µg each of isolated endolysin proteins were added to a bacterial cell suspension (addition indicated by the arrow, increasing protein concentration with increasing numbering of the traces) and decrease of the sample turbidity recorded until complete lysis.

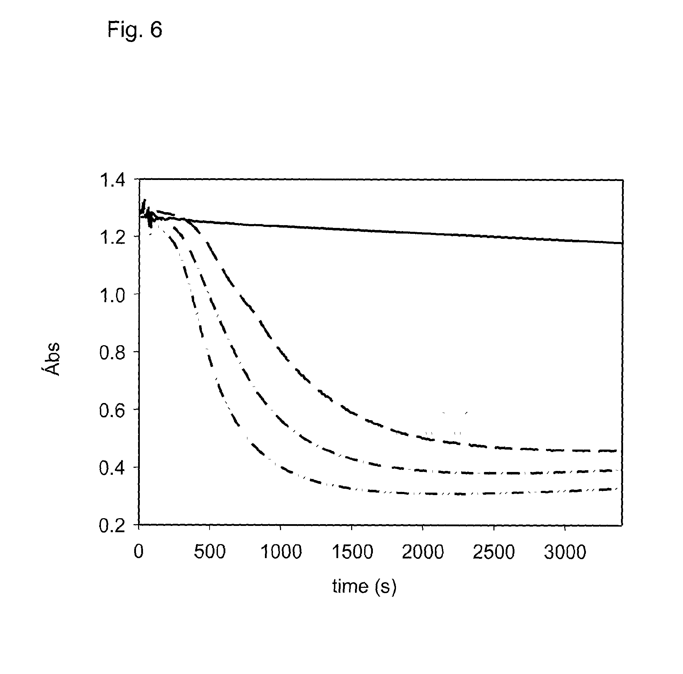

**Fig. 6** Lysis activity in human blood serum
   Fig. 6 shows lysis of *Staphylococcus aureus* cells by EADplypitti26_CBDplyUSA measured with the turbidity assay performed in human blood serum. The decrease in optical density (abs) is measured against assay time (s). EADplypitti26_CBDplyUSA at concentrations of 25 µg/ml (------), 50 µg/ml (-·-·-·-·-·) or 100 µg/ml (-··-··-··-) was applied at time point zero. The solid line represents a control without addition of endolysin. The assay is described in example 6.

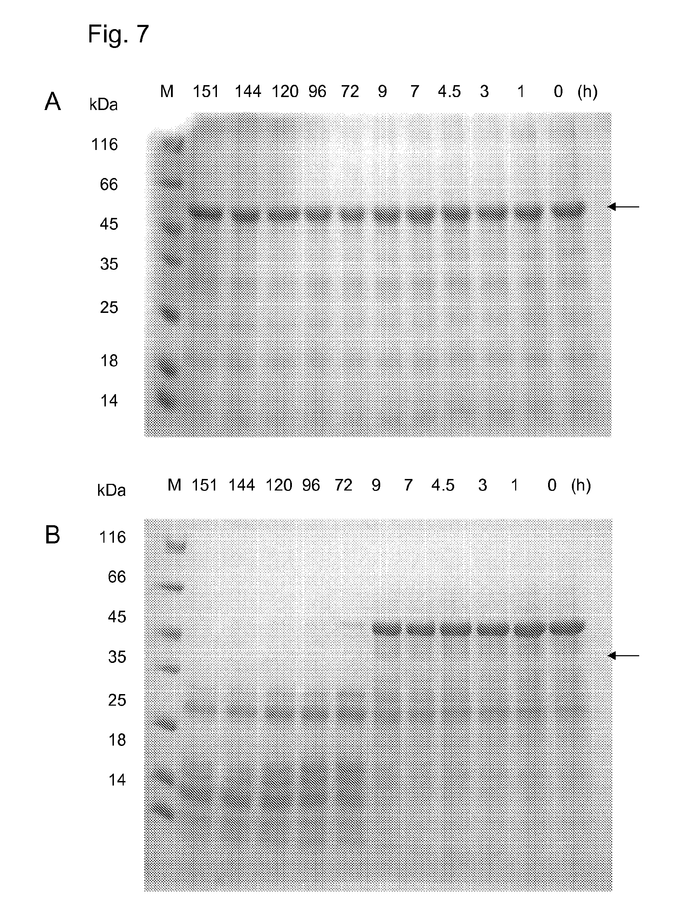

**Fig. 7** Stability of EADplypitti26_CBDplyUSA in comparison to wild-type pitti26
   The figure shows pictures of SDS-gels depicting endolysin preparations after incubation at 25°C in storage buffer (see example 6). In figure 7A, EADplypitti26_CBDplyUSA is applied onto the gel, figure 7B shows plypitti26 protein. The first lane represents a molecular standard with the time of incubation at 25°C in hours. The position of the full-length endolysin is marked by an arrow.

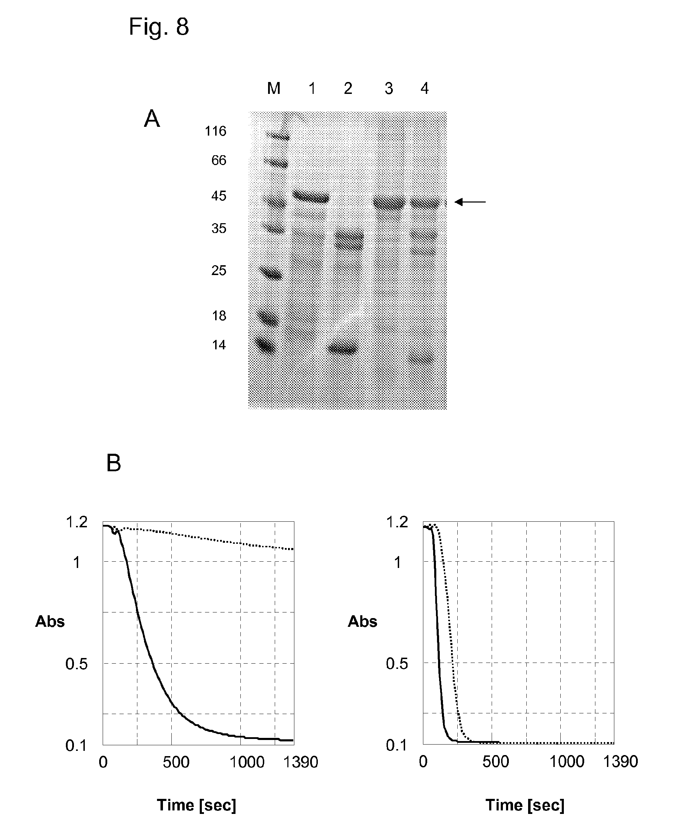

**Fig. 8** Stability against thrombin
   Figure 8A represents a picture of SDS-gels showing EADplypitti26_CBDplyUSA (lanes 3, 4) and wild-type plypitti26 (lanes 1, 2) before and after digestion with thrombin. The first lane (M) is a molecular mass standard. Lanes 1 and 3 are controls (without addition of thrombin), and lanes 2 and 4 show the protein samples after addition of thrombin. The position of the full-length endolysins is marked by an arrow. Figure 8B depicts an activity assay using turbidity measurements before and after digestion with thrombin. On the left plypitti26 is shown, on the right EADplypitti26_CBDplyUSA. Solid lines represent the lytic activity without addition of thrombin, dotted lines the residual activity after thrombin digestion. The experiment is described in example 8.

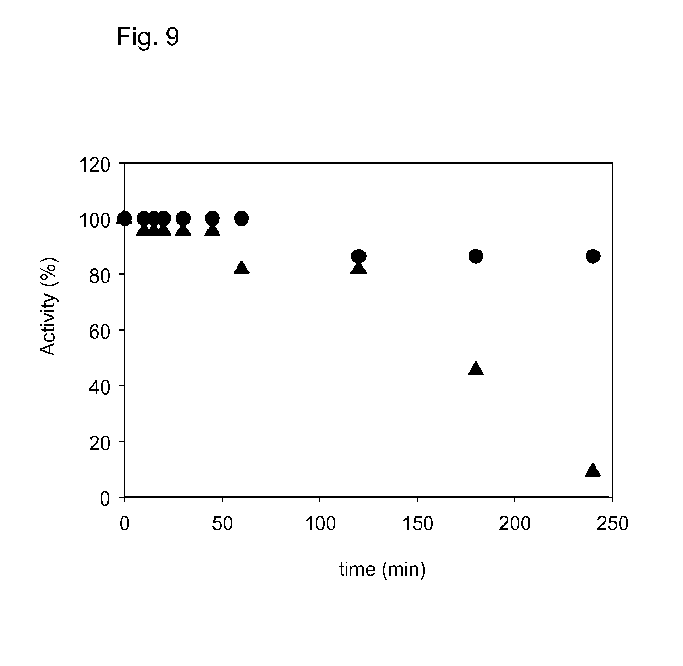

**Fig. 9** Stability in human blood
   Fig. 9 shows the stability of EADplypitti26_CBDplyUSA after preincubation in human blood at 37°C. The activity level achieved without preincubation is set to 100 %. (•) marks the activity after preincubation in buffer, and (▲) marks the respective preincubation in human EDTA-blood. The assays are described in example 9.

### Detailed Description of the Invention

The term "endolysin" as used herein refers to an enzyme which is suitable to hydrolyse bacterial cell walls. The enzyme comprises at least one of the following activities of which the "enzymatically active domains" (EADs) of the endolysins are constituted: endopeptidase, N-acetyl-nuramoyl-L-alanine-amidase (amidase), N-acetyl-muramidase or N-acetyl-glucosaminidase (lysozyme). Either, the enzyme is phage encoded or it is derived from related enzymes coded by bacteria, the so-called "autolysins". In addition, the endolysins usually contain also regions which are enzymatically inactive, and bind to the cell wall of the host bacteria, the so-called CBDs (cell wall binding domains).

The term "module" as used herein refers to a subunit of an endolysin which is ascribed a specific function. Generally, a module is a relatively small functional unit like CHAP-, ami- or SH3-modules.

The term "domain" as used herein refers to a subunit of an endolysin which is ascribed a specific function and can also coincide with structural domains. The term domain is preferentially used to describe the antagonism between EAD which can be composed of more than one module and CBD domains.

The term "CBD" as used herein refers to the cell wall binding domain of an endolysin, which is often found at the C-terminus of the protein. CBD domains have no enzymatic acitivity in terms of hydrolyzing the cell wall, but often mediate binding of the endolysin to the bacterial cell wall. CBD may contain an SH3-domain.

The term "EAD" as used herein refers to the enzymatically active domain of an endolysin which is responsible for hydrolysis of the bacterial peptideglycan. It contains at least one of the enzymatic activities of an endolysin. The EAD can also be composed of more than one enzymatically active module.

A "CHAP" domain (cysteine, histidine-dependent amidohydrolases/peptidases) is a region between 110 and 140 amino acids that is found in proteins from bacteria, bacteriphages, archaea and eukaryotes of the Trypanosomidae family. The proteins may function mainly in peptidoglycan hydrolysis. The CHAP domain is commonly associated with bacterial type SH3 domains and with several families of amidase domains. CHAP domain containing proteins may utilize a catalytic cysteine residue in a nucleophilic-attack mechanism. The CHAP domain contains two invariant amino acid residues, a cysteine and a histidine. These residues form part of the putative active site of CHAP domain containing proteins.

The term "ami" as used herein describes an enzymatically defined module which exhibits amidase activity, i.e. it hydrolyzes the amide bond between N-acetylmuramine in the peptidoglycan backbone and the adjacent amino acid which is usually L-ala in the peptide linker. The amidase are often metal ion dependent for activity.

The term "SH3" domain which is sometimes also called Src homology 3 domain as used herein describes a small non-catalytic protein domain of about 60 amino acids which is characteristic for proteins which interact with other binding partners. It is identified via a praline-rich consensus motif. The SH3 domain is usually located within the CBD.

The term "shuffling" as used herein refers to the combination of different fragments of polypeptides from different enzymes into new polypeptide constructs. In this context, the enzymes are preferentially endolysins, and the fragments are preferentially modules. Usually, the fragments are combined by molecular biological methods on nucleic acid level. Small linker sequences may be introduced between the fragments for structural or cloning reasons.

The inventors isolated several lytic bacteriophages from sewage samples which were active against Staphylococci using standard techniques for bacteriophage isolation (Adams, 1959, In: Bacteriophages, Interscience Pub., New York, p. 447-451). Several lysogenic phages were identified using molecular biological techniques within *Staphylococcus aureus* strains isolated from epidemic strains.

One isolated phage was named pitti26. Within the phage genomes of the isolated lysogenic phages endolysin proteins were identified and isolated. One preferred endolysin was isolated from the lytic phage pitti26 and was named ply_pitti26 (SEQ ID NO:1). A further endolysin was identified within the lytic phage pitti20 and named ply_pitti20. A prophage derived endolysin, plyUSA, was identied in the genome of the prophage ΦSA2usa which was integrated into the genome of the meticillin-resistant *Staphylococcus aureus* strain USA300 (Diep et al., The Lancet, 2006, 367, 731-739; databank entry NC_007793).

Endolysins of bacteriophages specific to *Staphylococcus* bacteria are typically composed of two enzymatically active domains, namely a CHAP domain, and an amidase domain (ami), and a cell binding domain (CBD) which often is defined as an SH3-domain of the SH3_b or SH3_5 type.

The inventor of the present invention combined the CHAP and the amidase domain of the endolysin plypitti26 with the cell binding domain of the prophage endolysin plyUSA and with the cell binding domain of the endolysin plypitti20 to generate a chimeric endolysin. The isolated endolysin plypitti26 as well as the chimeric enzymes can be used to lyse bacteria of the genus *staphylococcus.*

In one aspect the present invention relates to a polypeptide termed ply_pitti26 comprising the sequence as depicted in SEQ ID NO:1.

In a further aspect the present invention relates to variants of this polypeptide termed ply_pitti26 comprising the sequence as depicted in SEQ ID NO:1. The following embodiments are considered to be variants of ply_pitti26:
a) polypeptides comprising a sequence in which the CBD of ply_pitti26 has been replaced by a CBD domain of another *Staphlococci* specific endolysin,
b) polypeptides comprising the sequence of ply_pitti26 except for at least the first N-terminal and at most the first 28 N-terminal amino acids of ply_pitti26,
c) polypeptides comprising one or more point mutations in the sequence of ply_pitti26,
d) polypeptides comprising in addition to SEQ ID NO: 1 marker moieties, tags or other functional polypeptide sequences, and
e) any combinations of a), b), c) and d).

As mentioned above, the present invention relates to a polypeptide comprising the sequence of SEQ ID NO: 1 as well as variants thereof. Said polypeptide as well as variants thereof are considered to be the polypeptides according to the present invention. All of them share the common feature to function as endolysin and are thus capable to lyse Staphylococcus bacteria. Said specificity for *Staphylococci* can be tested by a plurality of methods which are known in the art, e.g. by adding the recombinant endolysin variant to a sample comprising one or more of said *Staphylococcus* species and determining the change in turbidity after addition of the (recombinant) endolysin.

In a preferred embodiment the variant of ply_pitti26 is a polypeptide wherein the CBD domain of the ply_pitti26 has been replaced by the CBD of another endolysin. In principle there is no restraint with regard to choosing other CBD domains from anti-staphylococcal endolysins as long as the resulting (recombinant) endolysin retains a specificity for *Staphylococcus* bacteria, in particular for *S. aureus, S. aureus (MRSA), S. epidermidis, S. haemolyticus, S. simulans, S. saprophyticus, S. chromogenes, S. hyicus, S. warneri* and/or *S. xylosus.* In a particularly preferred embodiment said recombinant endolysin comprises an endolysin cell binding domain of the SH3 type. Preferably, the CBD domains are selected from the endolysin CBD domains of ply_USA or ply_pitti20. As CBD of ply_USA in particular the sequence as denoted in SEQ ID NO: 3 is preferred. As CBD of ply_pitti20 in particular the sequence as denoted in SEQ ID NO: 5 is preferred. Illustrative examples for such recombinant endolysin variants of ply_pitti26 with exchanged CBD are given in SEQ ID NOs: 7 and 11.

In a further preferred embodiment the variant of ply_pitti26 comprises the sequence of ply_pitti 26 except for a certain number of N-terminal residues. The inventors of the present invention discovered, that if more than 28 N-terminal amino acid residues are removed from SEQ ID NO:1 then the endolysin activity is lost. Therefore, suitable variants of ply_pittii26 comprise in principle the sequence of SEQ ID NO:1 but lack 1, 2, 3, 4, 5, 6, 7, 8, 9, 10, 11 , 12, 13 , 14, 15 , 16 ,17 ,18, 19, 20, 21 ,22, 23, 24, 25, 26, 27 or 28 N-terminal amino acid residues of SEQ ID NO:1. Particularly preferred are variants which lack 4, 9 or 28 of the N-terminal amino acid residues of ply_pitti26.

In a preferred embodiment, the variants of the polypeptide termed ply_pitti26 exhibit single or multiple substitutions with regard to SEQ ID NO: 1. In particular, the sites to be substituted for other amino acid residues are F19, W22, W36, F42, Y44,155,156, F67, L74, Y78, W107, Y115, I116, Y119, W123, W128, W137, W139, W154, E163, R167, E179, E189, Y200, Y275, Y276, C282, F300 and/or C303. All positions are indicated with regard to SEQ ID NO: 1.

In an even more preferred embodiment hydrophobic amino acids such as F, W, Y, I, L are exchanged against less hydrophobic amino acids such as R, D, E, N, K, Q, H, S, T, M, G, A, wherein A is particularly preferred. Charged amino acids such as E and R are preferentially exchanged against uncharged amino acids (e.g. Q or A for E, A for R). Cysteines are preferentially exchanged against A or S.

Particularly preferred are variations in the sequence of SEQ ID No:1 selected from the following group of mutations. All substitutions are given with regard to the position in SEQ ID NO:1: W22R, F42A, F44A F67T, Y115S, W123M, W137A, W139A, W154H, E179Q, E179A, E187Q, Y200A , Y200H, Y275A , Y275M, Y276A , C282A , F300A, C303S, W310A and/or W310M. Said substitutions may be single mutants of SEQ ID NO:1 or may be a combination of two or more of said substitutions. Preferred multiple mutants of SEQ ID NO:1 are selected form the group comprising the following multiple mutants: F67T + Y115S, F67T + W137A, F67T + W139A, F67T + W154H, Y115S + W137A, Y115S + W139A, E163Q + R169A, E163A + R169A, E163Q + R167A + E189Q, E163A + R167A + E189Q, E163Q + R167A + E179Q + E189Q, E163Q + R167A + E179A + E189Q, E163A + R167A + E179Q + E189Q, E163A + R167A + E179A + E189Q, Y200A + Y275A, Y200A + Y276A, Y200A + C282A, Y200A + F300A, Y275A + Y276A.

In a further preferred embodiment the variant of ply_pitti26 comprises additional marker moieties such as biotin or Streptavidin or tags such as HA-tag, His-tag, Strep-tag, Myc-tag, GST-tag or other tags known in the art. Preferred other functional polypeptide sequences are for example protease cleavage sites. In some embodiments the above mentioned variants of ply_pitti26 may serve for facilitating the biotechnological production of ply_pitti26 or said variants.

It has to be understood that the above illustrated variants of ply_pitti26 must not be considered as strictly separate embodiments but instead may be combined. For example, a variant of ply_pitti26 according to the present invention may comprise a N-terminal truncation, of SEQ ID NO:1, one or more point mutations as indicated above, the CBD domain of another endolysin as well as for example a His-tag with the proviso that such recombinant endolysin is still specific for Staphylococci. The person skilled in the art will readily realize which of said variants is suitable for his purposes and can always test such variants for activity against Staphylococci by routine methods in the art, for example by assaying the effect of lysis activity on the optical density of a staphylococci containing solution. Illustrative Examples for such combinatorial variants are given in SEQ ID NO: 9 (ply_pitti26 with CBD of plyUSA and additional c-terminal His-tag) and SEQ ID NO: 13 (ply_pitti26 with CBD of ply_pitti20 and additional C-terminal His-tag).

In another aspect the present invention relates to a nucleic acid encoding one of the polypeptides of the invention. It is apparent to a person skilled in the art that there may be many ways of constructing nucleic acids encoding for one of the polypeptides according to the present invention, for example in view of the degenerate genetic code. A person skilled in the art with knowledge about the amino acid sequence of the polypeptide of the present invention is capable of choosing an adequate nucleic acid sequence which suits his purposes most, for example in which the codon usage has been adapted to the codon usage of his desired expression host. A nucleic acid comprising a sequence encoding for a polypeptide of the invention is considered to be the nucleic acid or nucleic acid sequence, respectively, of the present invention.

In a preferred embodiment said nucleic acid encodes for the endolysin comprising the sequence of SEQ ID NO: 1. Preferably, said nucleic acid comprises the nucleic acid sequence as depicted in SEQ ID NO: 2.

In another preferred embodiment the polypeptide to be encoded is a variant of ply_pitti26, in particular a variant wherein the CBD domain is replaced by a CBD of another Staphylococcal bacteriophage endolysin. Illustrative examples for nucleic acid sequences encoding the CBD of plyUSA or ply_pitti20 are given in SEQ ID NOs: 4 and 6, respectively. Further example illustrating nucleic acid sequences of the present invention are given in SEQ ID NOs: 8 (encoding for ply_pitti26 with CBD of ply_USA), 10 (encoding for ply_pitti26 with CBD of ply_USA plus additional c-terminal His-tag), 12 (encoding for ply_pitti26 with CBD of ply_pitti20) and 14 (encoding for ply_pitti26 with CBD of ply_pitti20 plus additional c-terminal His-tag), respectively.

In a further aspect the present invention relates to a vector comprising a nucleic acid sequence of the invention. Preferably, said vector provides for the expression of said polypeptide of the invention in a suitable host cell. Said host cell may be selected due to mere biotechnological reasons, e.g. yield, solubility, costs, etc. but may be also selected from a medical point of view, e.g. a non-pathological bacteria or yeast, human cells, if said cells are to be administered to a subject. Said vector may provide for the constitutive or inducible expression of said polypeptides according to the present invention.

In a further aspect of the present invention the above mentioned polypeptides and/or cells are employed in a method for the treatment or prophylaxis of *Staphylococci* infections in a subject, in particular for the treatment or prophylaxis of infections by *S. aureus, S. aureus (MRSA), S. epidermidis, S. haemolyticus, S. simulans, S. saprophyticus, S. chromogenes, S. hyicus, S. warneri* and/or *S. xylosus.* Said subject may be a human subject or an animal, in particular animals used in livestock farming and/or dairy farming such as cattle. Said method of treatment encompasses the application of said polypeptide of the present invention to the site of infection or site to be prophylactically treated against infection in a sufficient amount.

In particular said method of treatment may be for the treatment or prophylaxis of infections, in particular by *Staphylococcus aureus,* of the skin, of soft tissues, of bacteremia and/or endocarditis.

In a further preferred embodiment a polypeptide according to the present invention is employed in a method for the treatment of keratitis, in particular of keratitis caused by *S. haemolyticus*.

In a further preferred embodiment a polypeptide according to the present invention is used to treat or prevent endoplastitis, in particular endoplatitis caused by *S. epidermidis*.

In a further preferred embodiment a polypeptide according to the present invention is used to treat or prevent urinary tract infections, in particular endoplatitis caused by *S. saprophyticus*.

In a further preferred embodiment a polypeptide according to the present invention is used in a method of treatment (or prophylaxis) of staphylococcal infections in animals, in particular in livestock and dairy cattle. In particular a polypeptide of the present application is suitable for use in methods of treatment (or prophylaxis) of bovine mastitis, in particular of bovine mastitis caused by *S. aureus, S. epidermidis, S. simulans, S. chromogenes, S. hyicus, S. warneri* and *S. xylosus.*

Furthermore, a polypeptide of the present invention may be used prophylactically as sanitizing agent, in particular before or after surgery, or for example during hemodialysis. Similarly, premature infants and immunocompromised persons, or those subjects with need for prosthetic devices can be treated with a polypeptide of the present invention, either prophylactically or during acute infection. In the same context, nosocomial infections by *Staphylococcus,* in particular by *S.* aureus or *S. aureus (MRSA)*, may be treated prophylactically or during acute phase with a polypeptide of the present invention.

In a particularly preferred embodiment a polypeptide of the present invention is used for medical treatment, if the infection to be treated (or prevented) is caused by multiresistant *Staphylococcus* strains, in particular by strains resistant against vancomycin, linezolid or daptomycin. Furthermore, a polypeptide of the present invention can be used in methods of treatment by administering them in combination with conventional antibacterial agents, such as antibiotics, other endolysins, etc.

The dosage and route of administration used in a method of treatment (or prophylaxis) according to the present invention depends on the specific disease/site of infection to be treated. The route of administration may be for example in particular embodiments oral, topical, nasopharyngeal, parenteral, intravenous, rectal or any other route of administration.

For application of a polypeptide of the present invention to a site of infection (or site endangered to be infected) a polypeptide of the present invention may be formulated in such manner that the endolysin is protected from environmental influences such as proteases, oxidation, immune response etc., until it reaches the site of infection.

Therefore, a polypeptide of the present invention may be formulated as capsule, dragee, pill, suppository, injectable solution or any other medical reasonable galenic formulation. In some embodiments these galenic formulation may comprise suitable carriers, stabilizers, flavourings, buffers or other suitable reagents.

For example, for topical application a polypeptide of the present invention may be administered by way of a lotion or plaster.

For nasopharyngeal application a polypeptide according to the present invention may be formulated in saline in order to be applied via a spray to the nose.

For treatment of the intestine, for example in bovine mastitis, suppository formulation can be envisioned. Alternatively, oral administration may be considered. In this case, the polypeptide of the present invention has to be protected from the harsh digestive environment until the site of infection is reached. This can be accomplished for example by using bacteria as carrier, which survive the initial steps of digestion in the stomach and which secret later on a polypeptide of the present invention into the intestinal environment.

All medical applications rely on the effect of the polypeptides of the present invention to lyse specifically and immediately staphylococcal bacteria when encountered. This has an immediate impact on the health status of the treated subject by providing a reduction in pathogenic bacteria and bacterial load and simultaneously relieves the immune system. Thus, the mayor task a person skilled in the art faces is to formulate the polypeptides of the present invention accurately for the respective disease to be treated. For this purpose usually the same galenic formulation as employed for conventional medicaments for these applications can be used.

In a further aspect of the present invention the above mentioned polypeptides and/or cells are a component of a pharmaceutical composition, which optionally comprises a carrier substance.

In an even further aspect the polypeptides and/or cells are part of a cosmetics composition. As mentioned above, several staphylococcal species can cause irritations on environmentally exposed surfaces of the patient's body such as the skin. In order to prevent such irritations or in order to eliminate minor manifestations of said staphylococcal pathogens, special cosmetic preparations may be employed, which comprise sufficient amounts of polypeptides of the present invention in order to lyse already existing or freshly settling *Staphylococci*.

In a further aspect the present invention relates to the use of said polypeptides according to the present invention in foodstuff, on food processing equipment, in food processing plants, on surfaces coming into contact with foodstuff such as shelves and food deposit areas and in all other situations, where staphylococcal bacteria can potentially infest food material.

### Examples

All cloning procedures were performed using standard techniques according to Sambrook et al. (Molecular cloning. A laboratory manual; 2nd ed. Cold Spring Harbor Laboratory Press 1989). Mutations and deletions were also introduced using standard techniques.

### Example 1: Cloning of the endolysins according to the invention

For EADplypitti26_CBDplyUSA, the nucleotide sequence of the CHAP-Ami2 domain was amplified from the lytic phage pitti26 (own isolate) and the CBD sequence was amplified from plyUSA. For EADplypitti26_CBDplypitti20, the CBD was derived from plypitti20 (own isolate). The fragments were combined by ligation, and cloned into the expression vector pET14b *via* the restriction sites NcoI and BamHI. In order to add a C-terminal His-tag to the sequence, the new construct was cloned into the expression vector pQE60 *via* the restriction sites NcoI and BamHI. This construct contains an additional sequence of Ser-Arg-Ser-(His6) at the C-terminus.

### Example 2: Expression and solubility testing of the endolysins according to the invention

For expression of the cloned constructs E. *coli* HMS174(DE3) (pET14b construct) and E. *coli* M15 (pQE construct) were used, respectively. Cells were grown in LB medium containing ampicillin and rifampicin (HMS174(DE3)) or ampicillin (M15) at 30°C or 37 °C with shaking and induced with 1 mM IPTG at OD_{600 nm} 0.4-0.6 (mid log phase). After shaking for further 3-4 hours the cells were harvested by centrifugation and frozen at -20°C.
For solubility testing, harvested cells were resuspended in lysis buffer (20 mM Tris/HCl pH 8.0, 5 mM EDTA), sonicated (2 x 30 sec) and centrifuged. The pellet was solved in exactly the same volume of buffer as the supernatant. Identical sample volumes of pellet and supernatant fractions were analyzed with 12 % SDS-PAGE (Fig. 3 and 4).

Whereas upon expression at 37 °C the solubility of recombinantly produced endolysins was generally poor, the solubility of EADplypitti26_CBDplyUSA was significantly higher than that of wild-type plypitti26 at 30 °C. This is shown in figure 4. Whereas almost all protein was found in the insoluble pellet fraction with plypitti26 (Fig. 4A), a soluble fraction of around 30 % to 40 % was found upon expression of EADplypitti26_CBDplyUSA (Fig. 4B). This holds similar for EADplypitti26_CBDplypitti20.

### Example 3: Purification of the endolysins according to the invention

For the purification of the soluble His-tagged versions of the endolysin constructs according to the invention a Ni-NTA-Sepharose column (Amersham) was used according to the manufacturer's instructions. The cell pellet was resuspended in equilibration buffer (25 mM Tris/HCl pH 8.0, 500 mM NaCl, 20 mM imidazole, 0.1 % Tween 20, 10 % glycerol), centrifuged and added to the column equilibrated with the same buffer. Elution of the protein was carried out in elution buffer (25 mM Tris/HCl pH 8.0, 500 mM imidazole, 0.1 % Tween 20, 10 % glycerol), and the eluted fractions were analyzed with SDS-PAGE. The combined fractions containing purified endolysins were dialyzed against storage buffer (20 mM Tris/HCl pH 7.5, 10 mM DTE, 0.1 mM ZnSO₄) and stored at -20°C.

The purification of the soluble version of the endolysins according to the invention without His-tag followed standard purification procedures with anion exchange chromatography, size exclusion chromatography and hydrophobic chromatography using for example Streamline HST, Superdex, and HiTrap Capto MMC columns (GE Healthcare).

The amount of active endolysin can be increased if insoluble endolysin material deposited in inclusion bodies (pellet fraction of example 2) is solubilized under denaturing conditions and subsequently refolded. The amount of protein deposited in inclusion bodies can be increased by increasing expression temperature to 37 °C, for example. Suitable conditions for solubilization and refolding are described for example in Navarre et al., 1999, J. Biol. Chem. 274, 15847-15856.

### Example 4: Agar plate activity assay

An overnight culture of a *staphylococcus* strain which was growing for about 18 hours at 37°C in Brain Heart Infusion medium (Oxoid), was collected by centrifugation, the cell pellet was resuspended in 1x DPBS (Merck) thereby reducing the original volume by a factor of 100 and heat inactivated at 80°C for 20 minutes. The cells were sonicated and solved in LB topagar inclulding ampicillin and IPTG (0.7 % agar (Bacto); 100 µg/ml ampicillin (Sigma); 1 mM IPTG (Roth)), and used for the preparation of "lysis plates". These plates can be overlayed with protein solutions or solutions of *E. coli* harbouring plasmids which code for the desired proteins. A lysis zone on the plate is visible if the lytic protein is active respectively is expressed at least partly in soluble and active form. See also Fig. 2 (plate showing lysis zones)

The agar plate assay was used to test the lysis activity of EADplypitti26_CBDplyUSA in comparison with the wild-type form of pitti26 using different coagulase positive (*Staphylococcus aureus*) and coagulase negative (e. g. *S. epidermidis, S. haemolyticus, S. saprophyticus, S. simulans*) Staphylococcus strains. This was done in an agar plate lysis assay. Concentrated heat inactivated (e. g. 20 min at 80 °C) Staphylococcus cells are fixed in a top agar layer in a way that a dense bacterial lawn is achieved. Either isolated endolysin solution (5 µl to 10 µl) or E. coli transformants harbouring the respective endolysin containing plasmids are stippled onto the surface of the top agar layer. The plates are incubated at 30 °C for several hours (1 h to 12 h), and then checked for lysis zones around the endolysin spots. Normally, an increase in the lysis zone diameter also coincides with a clearing of the lysis zone indicating more efficient cell lysis. The results are depicted in table 1.

**Table 1: Host range for lysis by EADplypitti26_CBDplyUSA and plypitti26**

| **PROFOS culture collection Nr.** | **Origin** | **Staphylococcus species** | **plypitti26** | **EADplypitti26 _CBDplyUSA** |
|---|---|---|---|---|
| S462 | Clinical isolate | *S. aureus* | +++ | +++ |
| S460 | Clinical isolate | *S. aureus* | ++ | +++ |
| S1516 | Clinical isolate | *S. aureus* | +++ | +++ |
| S459 | Clinical isolate | *S. aureus* | +++ | +++ |
| S457 | Clinical isolate | *S. aureus* | +++ | +++ |
| S456 | Clinical isolate | *S. aureus* | +++ | +++ |
| S1519 | Clinical isolate | *S. aureus* | +++ | +++ |
| S463 | Clinical isolate | *S. aureus* | +++ | +++ |
| S1551 | DSMZ346 | *S. aureus* | ++ | +++ |
| S1517 | Clinical isolate | *S. aureus* | +++ | +++ |
| S458 | Clinical isolate | *S. aureus* | +++ | +++ |
| S1550 | DSMZ20231 | *S. aureus* | ++ | ++ |
| S467 | Clinical isolate | *S. aureus, MRSA* | + | +++ |
| S469 | Clinical isolate | *S. aureus, MRSA* | +++ | +++ |
| S468 | Clinical isolate | *S. aureus, MRSA* | +++ | +++ |
| S1573 | Own isolate, patient | *S. epidermidis* | - | +++ |
| S1508 | Clinical isolate | *S. epidermidis* | +++ | +++ |
| S1510 | Clinical isolate | *S. epidermidis* | +++ | +++ |
| S1546 | DSMZ20044 | *S. epidermidis* | + | +++ |
| S27 | Own isolate | *S. haemolyticus* | + | +++ |
| S1548 | DSMZ20228 | *S. haemolyticus* | + | +++ |
| S1509 | Clinical isolate | *S. haemolyticus* | - | +++ |
| S1549 | DSMZ20263 | *S. haemolyticus* | + | +++ |
| S1429 | Own isolate, food | *S. saprophyticus* | - | +++ |
| S1512 | Clinical isolate | *S. simulans* | ++ | +++ |

Table 1 depicts the host range for the EADplypitti26_CBDplyUSA compared to the wild-type endolysin Pitti26. Lysis activity on different staphylococcus strains was tested with the agar plate activity assay. Strong (+++), medium (++), weak (+) and no (-) lysis are defined as described in the legend of Fig. 2. The first column depicts the strain number corresponding to the PROFOS culture collection. The second column mentions the origin of the respective strain. "Clinical isolate" means a strain isolated from a patient with diagnosed staphylococcal infection. DSMZ numbers represent strains that can be ordered from the "Deutsche Sammlung für Mikroorganismen und Zellkulturen" (Braunschweig) the remaining being own isolates from our laboratory. The lysis assay demonstrated that the activity of EADplypitti26_CBDplyUSA is often better than the activity of the wild-type enzyme plypitti26, especially on the coagulase negative non S. aureus strains. This means also that EADplypitti26_CBDplyUSA has a broader host range compared to plypitti26. Coagulase negative staphylococci (species other than *Staphylococcus aureus*) are involved in infections in weakened and immune compromised persons, cause problems in biofilm formation in persons wearing indwelling devices or other implants, and are involved in mastitis. *S. saprophyticus* causes special problems in urinary tract infections.

### Example 5: Turbidity assay to control lysis activity

The second activity test we use for endolysin function is a turbidity test, where lysis of bacterial cells is measured "online" in a photometer. The absorption at 600 nm is a measure for the density of the cell culture and decreases upon cell lysis when the sample also becomes visibly clear. In contrast to the agar plate assay described above, this assay uses non-heat-treated bacterial cells, and is therefore more stringent and closer to reality in medical applications. Lysis of bacteria by the phage endolysins results in a drop of optical density (OD) which is measured by the turbidity assay. Target bacteria were grown in Brain Heart Infusion (Oxoid) until an OD₆₀₀ of around 0.8 is reached (exponential phase). The cells were harvested and resuspended in TBST buffer (20 mM Tris/HCl pH 7.5, 60 mM NaCl, 0.1 % Tween) plus 2 mM CaCl₂ to an OD₆₀₀ of 1. Small volumes of concentrated endolysin solutions are added to final protein concentrations as indicated in the figures. Changes in OD₆₀₀ were followed at 30 °C in a sample volume of 1 ml in a photometer (Jasco) until a stable baseline is reached. The addition of the test proteins occurred without interruption of the measurement. (See Fig. 3B, 5, 6, 8).

Different concentrations of isolated plypitti26 and EADplypitti26_CBDplyUSA endolysin were tested on *Staphylococcus aureus* cells. The results are depicted in figure 5. EADplypitti26_CBDplyUSA (traces 4, 5, 6 in figure 5) generally showed higher activity at the same protein concentration than plypitti26 (traces 1, 2, 3 in figure 5), again indicating superior properties compared to the naturally occurring endolysin. With respect to the relatively low protein concentrations used in the assay, complete lysis (indicated by no residual absorption) is achieved in a very short time spam of several minutes with EADplypitti26_CBDplyUSA. The test was also performed with protein concentrations as low as 1 µg, 0.5 µg and even 0.2 µg, resulting also in complete lysis over a longer period of time. The resulting specific activity, defined as ΔOD₆₀₀ per mg protein and minute, was calculated for EADplypitti26_CBDplyUSA as a value of ca. 50 units. Comparing this specific activity with state of the art full-length and truncated versions of phi11 endolysin, which are between 0.8 and 1.5 units (Donovan et al., 2006, FEMS Microbiol. Lett, 265, 133-139; figure 2b), EADplypitti26_CBDplyUSA turns out to be an extremely efficient staphylococcal endolysin. Even plypitti26 is significantly better than the phi11 endolysin.

### Example 6: Activity assay in blood serum

The activity of EADplypitti26_CBDplyUSA in blood serum was tested with the photometric turbidity assay according to example 5. Log phase *S. aureus* cells were resuspended in blood serum and subsequently lysis assays with different concentrations of EADplypitti26_CBDplyUSA were performed. This assay allows a direct measuring of lysis of *S. aureus* cells in human blood serum (Fig. 6). For efficient lysis in serum around 10fold the amount of protein is required in comparison to lysis in osmolytically optimized lysis buffer (compare Fig. 5). In addition, the kinetics of lysis are slower in blood serum than in buffer.

The activity of EADplypitti26_CBDplyUSA was tested in human blood serum. The results are shown in figure 6. It could be demonstrated that EADplypitti26_CBDplyUSA is also an efficient lysin on *S. aureus* cells under conditions existent in human plasma, albeit a somewhat higher protein concentration is needed than under optimized standard assay conditions, and the lysis is somewhat slower (compare figure 5).

### Example 7: Stability during long term incubation at room temperature

The stability of EADplypitti26_CBDplyUSA in comparison to plypitti26 was tested after incubation in storage buffer (20 mM Tris/HCl pH 7.5, 10 mM DTE, 0.1 mM ZnSO₄) at 25 °C for up to one week. Degradation of the proteins was monitored by SDS-PAGE (Fig. 7A and B) and in turbidity assays. Whereas EADplypitti26_CBDplyUSA was stable over the whole time period tested as no changes were observed (Fig. 7A), plypitti26 was obviously degraded at incubation times longer than 9 hours (Fig. 7B), and no full-lenth protein was visible after 120 h of incubation, but protein bands corresponding to smaller fragments became visible.

Remaining activity of the tested proteins was recorded using the turbidity assay under standard conditions (see example 5). In accordance with the stability data obtained by SDS-PAGE, EADplypitti26_CBDplyUSA shows a slower decrease in activity in comparison to plypitti26. EADplypitti26_CBDplyUSA remains fully active for a long time, whereas the activity of the plypitti26 drops significantly already after 4 hours.

This correlated with the decrease in activity, suggesting that only full-length endolysins were enzymatically active under these conditions. The experiment demonstrated, that especially EADplypitti26_CBDplyUSA is stable for reasonable periods of time even at room temperature. The stability extends a lot when the proteins are stored at lower temperatures, e.g. at 4 °C in a refrigerator or at temperatures of - 20 °C or - 80 °C in a deep freezer.

### Example 8: Protease stability against thrombin

For the protease stability assay 50µg thrombin were incubated over night at 25 °C with the respective proteins at a final concentration of 1.1 mg/ml. The incubation buffer was 20 mM Tris/HCl pH 7.5, 10 mM DTE, 0.1 mM ZnSO₄. On the next day, the protein samples were analyzed on SDS-gels and in the turbidity assay. Whereas only a small amount of EADplypitti26_CBDplyUSA is degraded during the time of the assay, no full-length protein of plypitti26 is left under the same conditions (see Fig. 8A). This means that EADplypitti26_CBDplyUSA is still highly active after incubation with thrombin whereas plypitti26 loses its activity completely (Fig. 8B). EADplypitti26_CBDplyUSA exhibits thrombin resistance as a property which renders it helpful for application in wounds or intravenous.

### Example 9: Stability in human blood

Activity was measured with the turbidity assay (example 5) after preincubation of EADplypitti26_CBDplyUSA at 37 °C for the times indicated in either storage buffer (20 mM Tris/HCl pH 7.5, 10 mM DTE, 0.1 mM ZnSO₄) or human EDTA-blood. Human blood samples are centrifuged to sediment red blood cells at the times indicated, and 100 µl of protein solution from the supernatant are added to start the turbidity assay. Up to 2 h of incubation time, there is almost identical activity measured after preincubation in buffer and in blood. EADplypitti26_CBDplyUSA is inactivated almost completely after 4 h incubation in blood at 37 °C, whereas there is 80 % residual activity measured with the control incubation (see fig. 9).

### Example 10: Modified endolysins

We have generated several modified chimeric endolysins starting from the sequences of EADplypitti26_CBDplyUSA and EADplypitti26_CBDplypitti20.

### N-terminal truncations

Several N-terminally truncated forms of EADplypitti26_CBDplyUSA were constructed and tested for activity in the agar plate assay. Two constructs that were N-terminally shortened by 4 and 9 amino acids, respectively, exhibited lytic activity in the plating assay, whereas a construct that was shortened by 29 amino acids did not exhibit activity any more.

### Site directed mutagenesis

In order to further stabilize and solubilize EADplypitti26_CBDplyUSA or EADplypitti26_CBDplypitti20, site directed mutations at selected positions within the amino acid sequence were performed using specific primers for single amino acid substitutions, and the activity of the respective single, double and triple mutants was tested using the agar plate activity assay. We substituted all hydrophobic amino acids (F, W, Y, I, L) against the less hydrophobic amino acids R, D, E, N, K, Q, H, S, T, M, G, A by standard site directed mutagenesis methods. The charged amino acids E and R are preferentially exchanged against uncharged amino acids (Q or A for E, A for R). The C are preferentially exchanged against A or S. The substitutions as listed in table 2 turned out to maintain the activity or to improve the properties of the endolysin as a therapeutic and prophylactic agent against staphylococcal infections.

**Table 2: Single, double and triple mutants of EADplypitti26_CBDplyUSA and EADplypitti26_CBDplypitti20 showing lytic activity against staphylococci**

| **Single amino acid mutations** | |
|---|---|
| **mutations** | **activity** |
| W22R | Yes |
| F42A | Yes |
| F44A | Yes |
| F67T | Yes |
| Y115S | Yes |
| W123M | Yes |
| W137A | Yes |
| W139A | Yes |
| W154H | Yes |
| E179Q | Yes |
| E179A | Yes |
| E187Q | Yes |
| Y200A | Yes |
| Y200H | Yes |
| Y275A | Yes |
| Y275M | Yes |
| Y276A | Yes |
| C282A | Yes |
| F300A | Yes |
| C303S | Yes |
| W310A | Yes |
| W310M | Yes |
| | |

| **Mutation of 2 to 4 residues** | |
|---|---|
| **Mutations** | **activity** |
| F67T + Y115S | yes |
| F67T + W137A | yes |
| F67T + W139A | yes |
| F67T + W154H | yes |
| Y115S + W137A | yes |
| Y115S + W139A | yes |
| E163Q + R169A | yes |
| E163A + R169A | yes |
| E163Q + R167A + E189Q | yes |
| E163A + R167A + E189Q | yes |
| E163Q + R167A + E179Q + E189Q | yes |
| E163Q + R167A + E179A + E189Q | yes |
| E163A + R167A + E179Q + E189Q | yes |
| E163A + R167A + E179A + E189Q | yes |
| Y200A + Y275A | yes |
| Y200A + Y276A | yes |
| Y200A + C282A | yes |
| Y200A + F300A | yes |
| Y275A + Y276A | yes |
| Y275A + F300A | yes |
| C282A + F300A | yes |
| Y200A + Y275A + Y276A | yes |
| Y275A + Y276A + F300A | yes |

## Claims

1. Polypeptide comprising the sequence as depicted in SEQ ID NO:1 or variant thereof, said variant being:
a) a polypeptide comprising a sequence in which the CBD of SEQ ID NO:1 has been replaced by a CBD domain of another *Staphlococci* specific endolysin,
b) a polypeptide comprising the sequence of SEQ ID NO:1 except for at least the first N-terminal amino acid and at most the first 28 N-terminal amino acids of SEQ ID NO:1,
c) a polypeptide comprising one or more point mutations in the sequence of SEQ ID NO:1,
d) a polypeptide comprising in addition to the sequence of SEQ ID NO: 1 sequences representing marker moieties, tags or other functional polypeptide sequences, or
e) a polypeptide comprising a polypeptide sequence representing any combination of variants a), b), c) and d).

2. Polypeptide according to claim 1, wherein the polypeptide lyses *S. aureus, S. aureus (MRSA), S. epidermidis, S. haemolyticus, S. simulans, S. saprophyticus, S. chromogenes, S. hyicus, S. warneri* and/or *S. xylosus.*

3. Polypeptide according to anyone of the preceding claims, wherein the variant of the polypeptide comprising the sequence as depicted in SEQ ID NO:1 comprises an endolysin cell binding domain of the SH3 type.

4. Polypeptide according to anyone of the preceding claims, wherein the variant of the polypeptide comprising the sequence as depicted in SEQ ID NO:1 comprises a CBD domain selected from the endolysin CBD domains ofply_USA or ply_pitti20.

5. Polypeptide according to claim 4, wherein the variant of the polypeptide comprising the sequence as depicted in SEQ ID NO:1 comprises a CBD domain as denoted in SEQ ID NOs: 3 or 5.

6. Polypeptide according to claim 5, comprising the sequence as denoted in SEQ ID NOs: 7 or 11.

7. Polypeptide according to anyone of the preceding claims, wherein the variant of the polypeptide comprising the sequence as depicted in SEQ ID NO:1 lacks 1, 2, 3, 4, 5, 6, 7, 8, 9 , 10, 11 , 12, 13 , 14, 15 , 16 ,17 ,18, 19, 20, 21 ,22, 23, 24, 25, 26, 27 or 28 N-terminal amino acid residues of SEQ ID NO:1.

8. Polypeptide according to anyone of the preceding claims, wherein the variant of the polypeptide comprising the sequence as depicted in SEQ ID NO:1 exhibits single or multiple substitutions with regard to SEQ ID NO: 1, wherein the substituted residues are selected from the following amino acid residues of SEQ ID NO:1: F19, W22, W36, F42, Y44, I55, I56, F67, L74, Y78, W107, Y115, I116, Y119, W123, W128, W137, W139, W154, E163, R167, E179, E189, Y200, Y275, Y276, C282, F300 and C303.

9. Polypeptide according to claim 8, wherein replacements of amino acids residues F, W, Y, I, L are exchanged for amino acids residues R, D, E, N, K, Q, H, S, T, M, G or A

10. Polypeptide according to claim 9 exhibiting with regard to the sequence of SEQ ID NO:1 one or more substitutions selected from the group comprising: W22R, F42A, F44A F67T, Y115S, W123M, W137A, W139A, W154H, E179Q, E179A, E187Q, Y200A , Y200H, Y275A , Y275M, Y276A , C282A , F300A, C303S, W310A and W310M.

11. Polypeptide according to claim 10, comprising one of the following substitutions with regard to SEQ ID NO:1: F67T + Y115S, F67T + W137A, F67T + W139A, F67T + W154H, Y115S + W137A, Y115S + W139A, E163Q + R169A, E163A + R169A, E163Q + R167A + E189Q, E163A + R167A + E189Q, E163Q + R167A + E179Q + E189Q, E163Q + R167A + E179A + E189Q, E163A + R167A + E179Q + E189Q, E163A + R167A + E179A + E189Q, Y200A + Y275A, Y200A + Y276A, Y200A + C282A, Y200A + F300A, Y275A + Y276A.

12. Polypeptide according to anyone of the preceding claims, wherein the polypeptide comprises biotin or Streptavidin as additional marker moiety.

13. Polypeptide according to anyone of claims 1 to 11, wherein the polypeptide further comprises a HA-tag, His-tag, Strep-tag, Myc-tag or GST-tag.

14. Polypeptide according to claim 13, wherein the polypeptide comprises the sequence as denoted in in SEQ ID NO: 9 or SEQ ID NO: 13.

15. Nucleic acid molecule comprising a sequence encoding for a polypeptide according to anyone of claims 1 to 14.

16. Nucleic acid molecule according to claim 15, wherein the nucleic acid molecule comprises a sequence selected from SEQ ID NOs:2, 4, 6, 8, 10, 12 or 14.

17. Vector comprising a nucleic acid according to claims 15 or 16.

18. Host cell comprising the nucleic acid molecule of claims 15 or 16 or the vector of claim 17.

19. The polypeptide according to anyone of claims 1 to 14 for use as a medicament.

20. The polypeptide of claim 19, wherein the polypeptide is administered along with a carrier.

21. Use of the polypeptide according to anyone of claims 1 to 14 for the treatment or prevention of Staphylococcus infections.

22. The use of claim 21, wherein the Staphylococcus infections results from infections by S. aureus, S. aureus (MRSA), S. epidermidis, S. haemolyticus, S. simulans, S. saprophyticus, S. chromogenes, S. hyicus, S. warneri and/or S. xylosus.

23. The use according to claim 21 or 22, wherein the Staphylococcus infection results in a disease state.

24. The use according to anyone of claims 21 to 23, wherein the disease is bacteremia, endocarditis, keratitis, endoplastitis or bovine mastitis.

25. The use according to anyone of claims 21 to 24, wherein the polypeptide is administered along with a carrier.

26. The use according to anyone of claims 21 to 25, wherein the polypeptide is administered topically, orally or by i.v. injection.

27. Sanitizing composition comprising a polypeptide according to any one of claims 1 to 14 or a host cell according to claim 18.

28. Cosmetical composition comprising a polypeptide according to any one of claims 1 to 14 or a host cell according to claim 18.

29. Pharmaceutical composition comprising a polypeptide according to any one of claims 1 to 14 or a host cell according to claim 18.

30. Use of the polypeptide according to anyone of claims 1 to 14 for the treatment or prevention of staphylococcal contamination of foodstuff, of food processing equipment, of food processing plants, or of surfaces coming into contact with foodstuff.


SEQUENCE LISTING

<110>  PROFOS AG

<120>  New proteins for use in human and animal Staphylococcus
       infections

<130>  PRO-028 EP

<140>  EP 07 114 785.4
<141>  2007-08-22

<160>  14

<170>  PatentIn version 3.3


<210>  1
<211>  490
<212>  PRT
<213>  unknown

<220>
<223>  pitti26

<400>  1
Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala

290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Ser Ala Trp Lys Arg Asn Lys
385                 390                 395                 400
Tyr Gly Thr Tyr Tyr Met Glu Glu Ser Ala Arg Phe Thr Asn Gly Asn
                405                 410                 415
Gln Pro Ile Thr Val Arg Lys Ile Gly Pro Phe Leu Ser Cys Pro Val
            420                 425                 430
Ala Tyr Gln Phe Gln Pro Gly Gly Tyr Cys Asp Tyr Thr Glu Val Met
        435                 440                 445
Leu Gln Asp Gly His Val Trp Val Gly Tyr Thr Trp Glu Gly Gln Arg
    450                 455                 460
Tyr Tyr Leu Pro Ile Arg Thr Trp Asn Gly Ser Ala Pro Pro Asn Gln
465                 470                 475                 480
Ile Leu Gly Asp Leu Trp Gly Glu Ile Ser
                485                 490


<210>  2
<211>  1473
<212>  DNA
<213>  unknown

<220>
<223>  pitti26

<220>
<221>  misc_feature
<222>  (1221)..(1221)
<223>  n is a, c, g, or t

<400>  2
atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240
acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa      420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcaa gtgcatggaa acgtaataaa    1200
tatggtactt actacatgga ngaaagtgct agattcacaa acggtaatca accaatcact    1260
gtaagaaaaa taggaccatt cttatcatgc ccggtagctt accaattcca acctggtgga    1320

tattgtgatt atacagaagt gatgttacaa gatggtcatg tttgggtagg atatacatgg   1380
gaggggcaac gttattactt gcctattaga acatggaatg gttctgcccc acctaatcag   1440
atattaggtg acttatgggg agaaatcagt tag                                1473

<210>  3
<211>  101
<212>  PRT
<213>  unknown

<220>
<223>  prophage SA2USA

<400>  3
Met Pro Pro Val Pro Ala Gly Tyr Thr Leu Asp Lys Asn Asn Val Pro
1               5                   10                  15
Tyr Lys Lys Glu Gln Gly Asn Tyr Thr Val Ala Asn Val Lys Gly Asn
            20                  25                  30
Asn Val Arg Asp Gly Tyr Ser Thr Asn Ser Arg Ile Thr Gly Val Leu
        35                  40                  45
Pro Asn Asn Thr Thr Ile Thr Tyr Asp Gly Ala Tyr Cys Ile Asn Gly
    50                  55                  60
Tyr Arg Trp Ile Thr Tyr Ile Ala Asn Ser Gly Gln Arg Arg Tyr Ile
65                  70                  75                  80
Ala Thr Gly Glu Val Asp Ile Ala Gly Asn Arg Ile Ser Ser Phe Gly
                85                  90                  95
Lys Phe Ser Ala Val
            100

<210>  4
<211>  303
<212>  DNA
<213>  unknown

<220>
<223>  prophage SA2USA

<400>  4
atgccaccag tgccagcagg ttatacactc gataagaata atgtccctta taaaaaagaa     60
caaggcaatt acacagtagc taatgttaaa ggtaataatg taagagacgg ttattcaact    120
aattcaagaa ttacaggggt attacccaac aacacaacaa ttacgtatga cggtgcatat    180
tgtattaatg gttatagatg gattacttat attgctaata gtggacaacg tcgctatatt    240
gcgaccggag aggtagacat agcaggcaac cgaataagca gttttggtaa gtttagtgca    300
gtt                                                                  303

<210>  5
<211>  96
<212>  PRT
<213>  unknown

<220>
<223>  pitti20

<400>  5
Met Lys Arg Lys Lys Pro Lys Gly Trp Ser Glu Asn Pro Tyr Gly Thr
1               5                   10                  15
Tyr Tyr Lys Lys Val Asp Lys Thr Phe Ile Val Gly Ser Glu Lys Ile
            20                  25                  30
Glu Thr Arg Ile Gly Ser Pro Phe Leu Ser Ala Pro Ser Gly Gly His
        35                  40                  45
Val Thr Pro Asn Gln Lys Met Thr Phe Asp Tyr Leu Ala Gln Gln Asp

    50                  55                  60
Gly Tyr Glu Trp Gly Gln Leu Glu Asn Asn Arg Gly Gln Gln Glu Phe
65                  70                  75                  80
Val Pro Ile Arg Pro Leu Ser Gln Lys Glu Tyr Trp Gly Ile Leu Lys
                85                  90                  95

<210>  6
<211>  288
<212>  DNA
<213>  unknown

<220>
<223>  pitti20

<400>  6
atgaaacgta aaaaacctaa aggttggagt gaaaatcctt atggtactta ctataaaaaa      60
gttgataaaa catttattgt aggttcagaa aaaattgaaa cacgaattgg ttcacctttc     120
ttatctgcac caagtggagg acacgtgaca ccaaatcaaa aaatgacctt tgactattta     180
gcacaacaag atggttatga atggggacaa cttgaaaata atagaggaca acaagaattt     240
gtaccaatta gaccattaag tcaaaaagaa tattggggta ttttaaaa                  288

<210>  7
<211>  496
<212>  PRT
<213>  artificial sequence

<220>
<223>  pitti26-prophage SA2USA

<400>  7
Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln

                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Glu Leu Met Pro Pro Val Pro
385                 390                 395                 400
Ala Gly Tyr Thr Leu Asp Lys Asn Asn Val Pro Tyr Lys Lys Glu Gln
                405                 410                 415
Gly Asn Tyr Thr Val Ala Asn Val Lys Gly Asn Asn Val Arg Asp Gly
            420                 425                 430
Tyr Ser Thr Asn Ser Arg Ile Thr Gly Val Leu Pro Asn Asn Thr Thr
        435                 440                 445
Ile Thr Tyr Asp Gly Ala Tyr Cys Ile Asn Gly Tyr Arg Trp Ile Thr
    450                 455                 460
Tyr Ile Ala Asn Ser Gly Gln Arg Arg Tyr Ile Ala Thr Gly Glu Val
465                 470                 475                 480
Asp Ile Ala Gly Asn Arg Ile Ser Ser Phe Gly Lys Phe Ser Ala Val
                485                 490                 495

<210>  8
<211>  1497
<212>  DNA
<213>  artificial sequence

<220>
<223>  pitti26-prophage SA2USA

<400>  8
atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240
acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa      420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcag agctcatgcc accagtgcca    1200
gcaggttata cactcgataa gaataatgtc ccttataaaa aagaacaagg caattacaca    1260

gtagctaatg ttaaaggtaa taatgtaaga gacggttatt caactaattc aagaattaca    1320
ggggtattac ccaacaacac aacaattacg tatgacggtg catattgtat taatggttat    1380
agatggatta cttatattgc taatagtgga caacgtcgct atattgcgac cggagaggta    1440
gacatagcag gcaaccgaat aagcagtttt ggtaagttta gtgcagtttg ataataa       1497

<210>  9
<211>  506
<212>  PRT
<213>  artificial sequence

<220>
<223>  pitti26-prophage SA2USA

<400>  9
Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380

Ser Ser Asn Thr Val Lys Pro Val Ala Glu Leu Met Pro Pro Val Pro
385                 390                 395                 400
Ala Gly Tyr Thr Leu Asp Lys Asn Asn Val Pro Tyr Lys Lys Glu Gln
                405                 410                 415
Gly Asn Tyr Thr Val Ala Asn Val Lys Gly Asn Asn Val Arg Asp Gly
            420                 425                 430
Tyr Ser Thr Asn Ser Arg Ile Thr Gly Val Leu Pro Asn Asn Thr Thr
        435                 440                 445
Ile Thr Tyr Asp Gly Ala Tyr Cys Ile Asn Gly Tyr Arg Trp Ile Thr
    450                 455                 460
Tyr Ile Ala Asn Ser Gly Gln Arg Arg Tyr Ile Ala Thr Gly Glu Val
465                 470                 475                 480
Asp Ile Ala Gly Asn Arg Ile Ser Ser Phe Gly Lys Phe Ser Ala Val
                485                 490                 495
Gly Ser Arg Ser His His His His His His
            500                 505

<210>  10
<211>  1521
<212>  DNA
<213>  artificial sequence

<220>
<223>  pitti26-prophage SA2USA

<400>  10
atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240
acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa     420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcag agctcatgcc accagtgcca    1200
gcaggttata cactcgataa gaataatgtc ccttataaaa aagaacaagg caattacaca    1260
gtagctaatg ttaaaggtaa taatgtaaga gacggttatt caactaattc aagaattaca    1320
ggggtattac ccaacaacac aacaattacg tatgacggtg catattgtat taatggttat    1380
agatggatta cttatattgc taatagtgga caacgtcgct atattgcgac cggagaggta    1440
gacatagcag gcaaccgaat aagcagtttt ggtaagttta gtgcagttgg atccagatct    1500
catcaccatc accatcacta a                                              1521

<210>  11
<211>  491
<212>  PRT
<213>  artificial sequence

<220>
<223>  pitti26-pitti20

<400>  11
Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Glu Leu Met Lys Arg Lys Lys
385                 390                 395                 400
Pro Lys Gly Trp Ser Glu Asn Pro Tyr Gly Thr Tyr Tyr Lys Lys Val
                405                 410                 415
Asp Lys Thr Phe Ile Val Gly Ser Glu Lys Ile Glu Thr Arg Ile Gly
            420                 425                 430
Ser Pro Phe Leu Ser Ala Pro Ser Gly Gly His Val Thr Pro Asn Gln
        435                 440                 445
Lys Met Thr Phe Asp Tyr Leu Ala Gln Gln Asp Gly Tyr Glu Trp Gly
    450                 455                 460
Gln Leu Glu Asn Asn Arg Gly Gln Gln Glu Phe Val Pro Ile Arg Pro
465                 470                 475                 480
Leu Ser Gln Lys Glu Tyr Trp Gly Ile Leu Lys
                485                 490

<210> 12
<211> 1476
<212> DNA
<213> artificial sequence

<220>
<223> pitti26-pitti20

<400> 12
atgagtatca tcatggaggt ggcgacaatg caagcaaaat taactaaaaa agagtttata 60
gagtggttga aaacttctga gggaaaacaa ttcaatgtgg acttatggta tggatttcaa 120
tgctttgatt atgccaatgc tggttggaaa gttttgtttg gattacttct gaaaggttta 180
ggtgcaaaag atataccatt tgcaaacaat ttcgatggac tagctactgt ataccaaaat 240
acaccggact ttttggcaca acccggcgac atggttgtat tcggtagcaa ttacggtgca 300
ggatacggac acgtagcatg ggtaattgaa gcaactttag attatatcat tgtatatgag 360
cagaattggc taggcggtgg ctggactgac agaatcgaac aacccggctg gggttgggaa 420
aaagttacaa gacgacaaca tgcttacgat ttccctatgt ggtttatccg tcctaacttc 480
aaaagcgaaa cagctccacg atcaatacaa tctcctacgc aagcatctaa aaaggaaaca 540
gctaagccac aacctaaagc ggtagaactt aaaattatca aagatgtggt taaaggttat 600
gaccttccta aacgtggtgg taatcctaag ggtatagtta ttcataacga cgcaggaagc 660
aaaggggcaa cagcagaagc gtatcgaaac ggattagtta acgcaccttc atcaagatta 720
gaagcgggta ttgcgcatag ttatgtatca ggtaacacag tgtggcaagc tttagatgaa 780
tcgcaagtag gttggcatac tgctaaccaa ttaggcaata aatattatta cggtattgaa 840
gtgtgtcaat caatgggagc ggataatgcg acgtttttaa aaaatgaaca ggcgactttc 900
caagaatgcg ctagattgtt gaaaaaatgg ggattaccag caaacagaaa tacaatcaga 960
ttacacaacg aattcacttc aacatcatgc ccacacagaa gctcagtatt gcacactggt 1020
tttgacccag taactcgcgg tctattgcca gaagacaagc ggttgcaact taaagactac 1080
tttatcaagc agattagggc gtacatggat ggtaaaatac cggttgccac tgtctctaat 1140
gagtcaagcg cttcaagtaa tacagttaaa ccagttgcag agctcatgaa acgtaaaaaa 1200
cctaaaggtt ggagtgaaaa tccttatggt acttactata aaaaagttga taaaacattt 1260
attgtaggtt cagaaaaaat tgaaacacga attggttcac ctttcttatc tgcaccaagt 1320
ggaggacacg tgacaccaaa tcaaaaaatg acctttgact atttagcaca acaagatggt 1380
tatgaatggg gacaacttga aaataataga ggacaacaag aatttgtacc aattagacca 1440
ttaagtcaaa aagaatattg ggtattttta aaatag 1476

<210> 13
<211> 501
<212> PRT
<213> artificial sequence

<220>
<223> pitti26-pitti20

<400> 13
Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125

Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Glu Leu Met Lys Arg Lys Lys
385                 390                 395                 400
Pro Lys Gly Trp Ser Glu Asn Pro Tyr Gly Thr Tyr Tyr Lys Lys Val
                405                 410                 415
Asp Lys Thr Phe Ile Val Gly Ser Glu Lys Ile Glu Thr Arg Ile Gly
            420                 425                 430
Ser Pro Phe Leu Ser Ala Pro Ser Gly Gly His Val Thr Pro Asn Gln
        435                 440                 445
Lys Met Thr Phe Asp Tyr Leu Ala Gln Gln Asp Gly Tyr Glu Trp Gly
    450                 455                 460
Gln Leu Glu Asn Asn Arg Gly Gln Gln Glu Phe Val Pro Ile Arg Pro
465                 470                 475                 480
Leu Ser Gln Lys Glu Tyr Trp Gly Ile Leu Lys Gly Ser Arg Ser His
                485                 490                 495
His His His His His
            500


<210>  14
<211>  1506
<212>  DNA
<213>  artificial sequence

<220>
<223>  pitti26-pitti20

<400>  14
atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240

acaccggact ttttggcaca acccggcgac atggttgtat tcggtagcaa ttacggtgca 300
ggatacggac acgtagcatg ggtaattgaa gcaactttag attatatcat tgtatatgag 360
cagaattggc taggcggtgg ctggactgac agaatcgaac aacccggctg gggttgggaa 420
aaagttacaa gacgacaaca tgcttacgat ttccctatgt ggtttatccg tcctaacttc 480
aaaagcgaaa cagctccacg atcaatacaa tctcctacgc aagcatctaa aaaggaaaca 540
gctaagccac aacctaaagc ggtagaactt aaaattatca aagatgtggt taaaggttat 600
gaccttccta aacgtggtgg taatcctaag ggtatagtta ttcataacga cgcaggaagc 660
aaaggggcaa cagcagaagc gtatcgaaac ggattagtta acgcaccttc atcaagatta 720
gaagcgggta ttgcgcatag ttatgtatca ggtaacacag tgtggcaagc tttagatgaa 780
tcgcaagtag gttggcatac tgctaaccaa ttaggcaata aatattatta cggtattgaa 840
gtgtgtcaat caatgggagc ggataatgcg acgtttttaa aaaatgaaca ggcgactttc 900
caagaatgcg ctagattgtt gaaaaaatgg ggattaccag caaacagaaa tacaatcaga 960
ttacacaacg aattcacttc aacatcatgc ccacacagaa gctcagtatt gcacactggt 1020
tttgacccag taactcgcgg tctattgcca gaagacaagc ggttgcaact taaagactac 1080
tttatcaagc agattagggc gtacatggat ggtaaaatac cggttgccac tgtctctaat 1140
gagtcaagcg cttcaagtaa tacagttaaa ccagttgcag agctcatgaa acgtaaaaaa 1200
cctaaaggtt ggagtgaaaa tccttatggt acttactata aaaaagttga taaaacattt 1260
attgtaggtt cagaaaaaat tgaaacacga attggttcac ctttcttatc tgcaccaagt 1320
ggaggacacg tgacaccaaa tcaaaaaatg acctttgact atttagcaca acaagatggt 1380
tatgaatggg gacaacttga aaataataga ggacaacaag aatttgtacc aattagacca 1440
ttaagtcaaa aagaatattg ggtattttta aaaggatcca gatctcatca ccatcaccat 1500
cactaa 1506

SEQUENCE LISTING

<110>  PROFOS AG

<120>  New proteins for use in human and animal Staphylococcus
       infections

<130>  PRO-028 EP

<140>  unknown
<141>  2007-08-22

<160>  14

<170>  PatentIn version 3.3


<210>  1
<211>  490
<212>  PRT
<213>  pitti26

<400>  1

Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala

290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Ser Ala Trp Lys Arg Asn Lys
385                 390                 395                 400
Tyr Gly Thr Tyr Tyr Met Glu Glu Ser Ala Arg Phe Thr Asn Gly Asn
                405                 410                 415
Gln Pro Ile Thr Val Arg Lys Ile Gly Pro Phe Leu Ser Cys Pro Val
            420                 425                 430
Ala Tyr Gln Phe Gln Pro Gly Gly Tyr Cys Asp Tyr Thr Glu Val Met
        435                 440                 445
Leu Gln Asp Gly His Val Trp Val Gly Tyr Thr Trp Glu Gly Gln Arg
    450                 455                 460
Tyr Tyr Leu Pro Ile Arg Thr Trp Asn Gly Ser Ala Pro Pro Asn Gln
465                 470                 475                 480
Ile Leu Gly Asp Leu Trp Gly Glu Ile Ser
                485                 490


<210>  2
<211>  1473
<212>  DNA
<213>  pitti26

<220>
<221>  misc_feature
<222>  (1221)..(1221)
<223>  n is a, c, g, or t

<400>  2

atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240
acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa      420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcaa gtgcatggaa acgtaataaa    1200
tatggtactt actacatgga ngaaagtgct agattcacaa acggtaatca accaatcact    1260
gtaagaaaaa taggaccatt cttatcatgc ccggtagctt accaattcca acctggtgga    1320
tattgtgatt atacagaagt gatgttacaa gatggtcatg tttgggtagg atatacatgg    1380

gaggggcaac gttattactt gcctattaga acatggaatg gttctgcccc acctaatcag    1440
atattaggtg acttatgggg agaaatcagt tag                                 1473

<210>  3
<211>  101
<212>  PRT
<213>  prophage SA2USA

<400>  3

Met Pro Pro Val Pro Ala Gly Tyr Thr Leu Asp Lys Asn Asn Val Pro
1               5                   10                  15
Tyr Lys Lys Glu Gln Gly Asn Tyr Thr Val Ala Asn Val Lys Gly Asn
            20                  25                  30
Asn Val Arg Asp Gly Tyr Ser Thr Asn Ser Arg Ile Thr Gly Val Leu
        35                  40                  45
Pro Asn Asn Thr Thr Ile Thr Tyr Asp Gly Ala Tyr Cys Ile Asn Gly
    50                  55                  60
Tyr Arg Trp Ile Thr Tyr Ile Ala Asn Ser Gly Gln Arg Arg Tyr Ile
65                  70                  75                  80
Ala Thr Gly Glu Val Asp Ile Ala Gly Asn Arg Ile Ser Ser Phe Gly
                85                  90                  95
Lys Phe Ser Ala Val
            100

<210>  4
<211>  303
<212>  DNA
<213>  prophage SA2USA

<400>  4

atgccaccag tgccagcagg ttatacactc gataagaata atgtccctta taaaaaagaa      60
caaggcaatt acacagtagc taatgttaaa ggtaataatg taagagacgg ttattcaact     120
aattcaagaa ttacaggggt attacccaac aacacaacaa ttacgtatga cggtgcatat     180
tgtattaatg gttatagatg gattacttat attgctaata gtggacaacg tcgctatatt     240
gcgaccggag aggtagacat agcaggcaac cgaataagca gttttggtaa gtttagtgca     300
gtt                                                                   303

<210>  5
<211>  96
<212>  PRT
<213>  pitti20

<400>  5

Met Lys Arg Lys Lys Pro Lys Gly Trp Ser Glu Asn Pro Tyr Gly Thr
1               5                   10                  15
Tyr Tyr Lys Lys Val Asp Lys Thr Phe Ile Val Gly Ser Glu Lys Ile
            20                  25                  30
Glu Thr Arg Ile Gly Ser Pro Phe Leu Ser Ala Pro Ser Gly Gly His
        35                  40                  45
Val Thr Pro Asn Gln Lys Met Thr Phe Asp Tyr Leu Ala Gln Gln Asp
    50                  55                  60
Gly Tyr Glu Trp Gly Gln Leu Glu Asn Asn Arg Gly Gln Gln Glu Phe
65                  70                  75                  80
Val Pro Ile Arg Pro Leu Ser Gln Lys Glu Tyr Trp Gly Ile Leu Lys
                85                  90                  95

<210>  6
<211>  288
<212>  DNA
<213>  pitti20

<400>  6

atgaaacgta aaaaacctaa aggttggagt gaaaatcctt atggtactta ctataaaaaa      60
gttgataaaa catttattgt aggttcagaa aaaattgaaa cacgaattgg ttcacctttc     120
ttatctgcac caagtggagg acacgtgaca ccaaatcaaa aaatgacctt tgactattta     180
gcacaacaag atggttatga atggggacaa cttgaaaata atagaggaca acaagaattt     240
gtaccaatta gaccattaag tcaaaaagaa tattggggta ttttaaaa                  288


<210>  7
<211>  496
<212>  PRT
<213>  pitti26-prophage SA2USA

<400>  7

Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320

Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Glu Leu Met Pro Pro Val Pro
385                 390                 395                 400
Ala Gly Tyr Thr Leu Asp Lys Asn Asn Val Pro Tyr Lys Lys Glu Gln
                405                 410                 415
Gly Asn Tyr Thr Val Ala Asn Val Lys Gly Asn Asn Val Arg Asp Gly
            420                 425                 430
Tyr Ser Thr Asn Ser Arg Ile Thr Gly Val Leu Pro Asn Asn Thr Thr
        435                 440                 445
Ile Thr Tyr Asp Gly Ala Tyr Cys Ile Asn Gly Tyr Arg Trp Ile Thr
    450                 455                 460
Tyr Ile Ala Asn Ser Gly Gln Arg Arg Tyr Ile Ala Thr Gly Glu Val
465                 470                 475                 480
Asp Ile Ala Gly Asn Arg Ile Ser Ser Phe Gly Lys Phe Ser Ala Val
                485                 490                 495

<210>  8
<211>  1497
<212>  DNA
<213>  pitti26-prophage SA2USA

<400>  8

atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240
acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa     420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcag agctcatgcc accagtgcca    1200
gcaggttata cactcgataa gaataatgtc ccttataaaa aagaacaagg caattacaca    1260
gtagctaatg ttaaaggtaa taatgtaaga gacggttatt caactaattc aagaattaca    1320
ggggtattac ccaacaacac aacaattacg tatgacggtg catattgtat taatggttat    1380
agatggatta cttatattgc taatagtgga caacgtcgct atattgcgac cggagaggta    1440
gacatagcag gcaaccgaat aagcagtttt ggtaagttta gtgcagtttg ataataa       1497

<210>  9
<211>  506
<212>  PRT
<213>  pitti26-prophage SA2USA

<400> 9

Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Glu Leu Met Pro Pro Val Pro
385                 390                 395                 400
Ala Gly Tyr Thr Leu Asp Lys Asn Asn Val Pro Tyr Lys Lys Glu Gln
                405                 410                 415
Gly Asn Tyr Thr Val Ala Asn Val Lys Gly Asn Asn Val Arg Asp Gly
            420                 425                 430
Tyr Ser Thr Asn Ser Arg Ile Thr Gly Val Leu Pro Asn Asn Thr Thr
        435                 440                 445
Ile Thr Tyr Asp Gly Ala Tyr Cys Ile Asn Gly Tyr Arg Trp Ile Thr
    450                 455                 460
Tyr Ile Ala Asn Ser Gly Gln Arg Arg Tyr Ile Ala Thr Gly Glu Val

465                 470                 475                 480
Asp Ile Ala Gly Asn Arg Ile Ser Ser Phe Gly Lys Phe Ser Ala Val
                485                 490                 495
Gly Ser Arg Ser His His His His His His
            500                 505

<210>  10
<211>  1521
<212>  DNA
<213>  pitti26-prophage SA2USA

<400>  10

atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240
acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa     420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcag agctcatgcc accagtgcca    1200
gcaggttata cactcgataa gaataatgtc ccttataaaa aagaacaagg caattacaca    1260
gtagctaatg ttaaaggtaa taatgtaaga gacggttatt caactaattc aagaattaca    1320
ggggtattac ccaacaacac aacaattacg tatgacggtg catattgtat taatggttat    1380
agatggatta cttatattgc taatagtgga caacgtcgct atattgcgac cggagaggta    1440
gacatagcag gcaaccgaat aagcagtttt ggtaagttta gtgcagttgg atccagatct    1500
catcaccatc accatcacta a                                              1521

<210>  11
<211>  491
<212>  PRT
<213>  pitti26-pitti20

<400>  11

Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr

                100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255
Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Glu Leu Met Lys Arg Lys Lys
385                 390                 395                 400
Pro Lys Gly Trp Ser Glu Asn Pro Tyr Gly Thr Tyr Tyr Lys Lys Val
                405                 410                 415
Asp Lys Thr Phe Ile Val Gly Ser Glu Lys Ile Glu Thr Arg Ile Gly
            420                 425                 430
Ser Pro Phe Leu Ser Ala Pro Ser Gly Gly His Val Thr Pro Asn Gln
        435                 440                 445
Lys Met Thr Phe Asp Tyr Leu Ala Gln Gln Asp Gly Tyr Glu Trp Gly
    450                 455                 460
Gln Leu Glu Asn Asn Arg Gly Gln Gln Glu Phe Val Pro Ile Arg Pro
465                 470                 475                 480
Leu Ser Gln Lys Glu Tyr Trp Gly Ile Leu Lys
                485                 490

<210>  12
<211>  1476
<212>  DNA
<213>  pitti26-pitti20

<400>  12

atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240

acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa     420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcag agctcatgaa acgtaaaaaa    1200
cctaaaggtt ggagtgaaaa tccttatggt acttactata aaaaagttga taaaacattt    1260
attgtaggtt cagaaaaaat tgaaacacga attggttcac ctttcttatc tgcaccaagt    1320
ggaggacacg tgacaccaaa tcaaaaaatg acctttgact atttagcaca acaagatggt    1380
tatgaatggg gacaacttga aaataataga ggacaacaag aatttgtacc aattagacca    1440
ttaagtcaaa aagaatattg gggtatttta aaatag                              1476

<210>  13
<211>  501
<212>  PRT
<213>  pitti26-pitti20

<400>  13

Met Ser Ile Ile Met Glu Val Ala Thr Met Gln Ala Lys Leu Thr Lys
1               5                   10                  15
Lys Glu Phe Ile Glu Trp Leu Lys Thr Ser Glu Gly Lys Gln Phe Asn
            20                  25                  30
Val Asp Leu Trp Tyr Gly Phe Gln Cys Phe Asp Tyr Ala Asn Ala Gly
        35                  40                  45
Trp Lys Val Leu Phe Gly Leu Leu Leu Lys Gly Leu Gly Ala Lys Asp
    50                  55                  60
Ile Pro Phe Ala Asn Asn Phe Asp Gly Leu Ala Thr Val Tyr Gln Asn
65                  70                  75                  80
Thr Pro Asp Phe Leu Ala Gln Pro Gly Asp Met Val Val Phe Gly Ser
                85                  90                  95
Asn Tyr Gly Ala Gly Tyr Gly His Val Ala Trp Val Ile Glu Ala Thr
            100                 105                 110
Leu Asp Tyr Ile Ile Val Tyr Glu Gln Asn Trp Leu Gly Gly Gly Trp
        115                 120                 125
Thr Asp Arg Ile Glu Gln Pro Gly Trp Gly Trp Glu Lys Val Thr Arg
    130                 135                 140
Arg Gln His Ala Tyr Asp Phe Pro Met Trp Phe Ile Arg Pro Asn Phe
145                 150                 155                 160
Lys Ser Glu Thr Ala Pro Arg Ser Ile Gln Ser Pro Thr Gln Ala Ser
                165                 170                 175
Lys Lys Glu Thr Ala Lys Pro Gln Pro Lys Ala Val Glu Leu Lys Ile
            180                 185                 190
Ile Lys Asp Val Val Lys Gly Tyr Asp Leu Pro Lys Arg Gly Gly Asn
        195                 200                 205
Pro Lys Gly Ile Val Ile His Asn Asp Ala Gly Ser Lys Gly Ala Thr
    210                 215                 220
Ala Glu Ala Tyr Arg Asn Gly Leu Val Asn Ala Pro Ser Ser Arg Leu
225                 230                 235                 240
Glu Ala Gly Ile Ala His Ser Tyr Val Ser Gly Asn Thr Val Trp Gln
                245                 250                 255

Ala Leu Asp Glu Ser Gln Val Gly Trp His Thr Ala Asn Gln Leu Gly
            260                 265                 270
Asn Lys Tyr Tyr Tyr Gly Ile Glu Val Cys Gln Ser Met Gly Ala Asp
        275                 280                 285
Asn Ala Thr Phe Leu Lys Asn Glu Gln Ala Thr Phe Gln Glu Cys Ala
    290                 295                 300
Arg Leu Leu Lys Lys Trp Gly Leu Pro Ala Asn Arg Asn Thr Ile Arg
305                 310                 315                 320
Leu His Asn Glu Phe Thr Ser Thr Ser Cys Pro His Arg Ser Ser Val
                325                 330                 335
Leu His Thr Gly Phe Asp Pro Val Thr Arg Gly Leu Leu Pro Glu Asp
            340                 345                 350
Lys Arg Leu Gln Leu Lys Asp Tyr Phe Ile Lys Gln Ile Arg Ala Tyr
        355                 360                 365
Met Asp Gly Lys Ile Pro Val Ala Thr Val Ser Asn Glu Ser Ser Ala
    370                 375                 380
Ser Ser Asn Thr Val Lys Pro Val Ala Glu Leu Met Lys Arg Lys Lys
385                 390                 395                 400
Pro Lys Gly Trp Ser Glu Asn Pro Tyr Gly Thr Tyr Tyr Lys Lys Val
                405                 410                 415
Asp Lys Thr Phe Ile Val Gly Ser Glu Lys Ile Glu Thr Arg Ile Gly
            420                 425                 430
Ser Pro Phe Leu Ser Ala Pro Ser Gly Gly His Val Thr Pro Asn Gln
        435                 440                 445
Lys Met Thr Phe Asp Tyr Leu Ala Gln Gln Asp Gly Tyr Glu Trp Gly
    450                 455                 460
Gln Leu Glu Asn Asn Arg Gly Gln Gln Glu Phe Val Pro Ile Arg Pro
465                 470                 475                 480
Leu Ser Gln Lys Glu Tyr Trp Gly Ile Leu Lys Gly Ser Arg Ser His
                485                 490                 495
His His His His His
            500

<210>  14
<211>  1506
<212>  DNA
<213>  pitti26-pitti20

<400>  14

atgagtatca tcatggaggt ggcgacaatg caagcaaaat taactaaaaa agagtttata      60
gagtggttga aaacttctga gggaaaacaa ttcaatgtgg acttatggta tggatttcaa     120
tgctttgatt atgccaatgc tggttggaaa gttttgtttg gattacttct gaaaggttta     180
ggtgcaaaag atataccatt tgcaaacaat ttcgatggac tagctactgt ataccaaaat     240
acaccggact ttttggcaca acccggcgac atggttgtat tcggtagcaa ttacggtgca     300
ggatacggac acgtagcatg ggtaattgaa gcaactttag attatatcat tgtatatgag     360
cagaattggc taggcggtgg ctggactgac agaatcgaac aacccggctg gggttgggaa     420
aaagttacaa gacgacaaca tgcttacgat ttccctatgt ggtttatccg tcctaacttc     480
aaaagcgaaa cagctccacg atcaatacaa tctcctacgc aagcatctaa aaaggaaaca     540
gctaagccac aacctaaagc ggtagaactt aaaattatca aagatgtggt taaaggttat     600
gaccttccta aacgtggtgg taatcctaag ggtatagtta ttcataacga cgcaggaagc     660
aaaggggcaa cagcagaagc gtatcgaaac ggattagtta acgcaccttc atcaagatta     720
gaagcgggta ttgcgcatag ttatgtatca ggtaacacag tgtggcaagc tttagatgaa     780
tcgcaagtag gttggcatac tgctaaccaa ttaggcaata aatattatta cggtattgaa     840
gtgtgtcaat caatgggagc ggataatgcg acgtttttaa aaaatgaaca ggcgactttc     900
caagaatgcg ctagattgtt gaaaaaatgg ggattaccag caaacagaaa tacaatcaga     960
ttacacaacg aattcacttc aacatcatgc ccacacagaa gctcagtatt gcacactggt    1020
tttgacccag taactcgcgg tctattgcca gaagacaagc ggttgcaact taaagactac    1080
tttatcaagc agattagggc gtacatggat ggtaaaatac cggttgccac tgtctctaat    1140
gagtcaagcg cttcaagtaa tacagttaaa ccagttgcag agctcatgaa acgtaaaaaa    1200
cctaaaggtt ggagtgaaaa tccttatggt acttactata aaaaagttga taaaacattt    1260

attgtaggtt cagaaaaaat tgaaacacga attggttcac ctttcttatc tgcaccaagt 1320
ggaggacacg tgacaccaaa tcaaaaaatg acctttgact atttagcaca acaagatggt 1380
tatgaatggg gacaacttga aaataataga ggacaacaag aatttgtacc aattagacca 1440
ttaagtcaaa aagaatattg ggtattttta aaaggatcca gatctcatca ccatcaccat 1500
cactaa 1506